# EUROPEAN PATENT APPLICATION

(11) **EP 4 775 986 A1**
(43) Date of publication of application: **15.07.2026**
(21) Application number: 26151913.6
(22) Date of filing: 14.01.2026
(51) Int. Cl.: G01N 33/50, B01L 3/00, C07K 16/00

(54) **METHODS FOR ENRICHMENT AND HIGH-THROUGHPUT MICROFLUIDIC SCREENING OF ANTIGEN-SPECIFIC ANTIBODY-SECRETING CELLS**

(30) Priority: 15.01.2025 CN 202510063594; 14.01.2025 CN 202510057037
(71) Applicant: Shenzhen Dapu Biotechnology Co., Shenzhen City, Guangdong 518000 (CN)
(72) Inventor: SONG, Weihong, Shenzhen, 518000 (CN); YUAN, Xin, Shenzhen, 518000 (CN); LI, Ouyang, Shenzhen, 518000 (CN); LIAO, Kuanzhen, Guangdong, 518000 (CN); SONG, Ruyuan, Guangdong, 518000 (CN); XU, Xiaonan, Shenzhen, 518000 (CN)
(74) Representative: K&L Gates LLP

(57) **Abstract**

The present disclosure provides a universal, high-throughput antibody discovery platform based on: (i) a negative-selection method for enriching B cells or plasma B cells without relying on plasma B cell surface biomarkers, and (ii) a microfluidic droplet-based functional screening system capable of identifying single cells secreting antigen-specific antibodies. The enrichment method employs a customizable cocktail of antibodies that target non-B lineage immune cells, followed by magnetic bead depletion. Enriched antibody-secreting cells are subsequently encapsulated into picoliter or nanoliter droplets together with (a) antigen-coated beads, (b) a fluorescent labeled antigen with immunoglobulin-capture antibody-coated beads, or (c) antigen-expressing reporter cells, allowing for functional detection of secreted antibodies inside each droplet. Through multi-channel fluorescence detection-incorporating detection antibody signals, dead-cell exclusion dyes, and optional blocking-antibody controls-droplets containing antigen-specific antibody-secreting cells are isolated. The recovered cells undergo single-cell BCR amplification or bulk BCR sequencing to obtain naturally paired heavy and light chain sequences.

## Description

### CROSS REFERENCE TO RELATED APPLICATIONS

This application claims the benefit of Chinese Application No. 202510057037.6, filed January 14, 2025, and Chinese Application No. 202510063594.9, filed January 15, 2025. The contents of the aforementioned applications are hereby incorporated by reference in their entirety.

### TECHNICAL FIELD

The disclosure relates to immunology, antibody discovery, microfluidics, and single-cell functional screening. More specifically, it relates to enrichment of B cells and plasma B cells from a heterogeneous population regardless of species; single-cell encapsulation within microfluidic droplets; detection of secreted antigen-specific antibodies in droplets; high-throughput sorting of functional antibody-secreting cells; and recovery and sequencing of naturally paired immunoglobulin variable regions. The disclosure is applicable to monoclonal antibody discovery, diagnostic antibody development, repertoire analysis, and single-cell immune profiling, among others.

### BACKGROUND

Monoclonal secreting antibodies are antibodies produced by a single B-cell clone that can be continuously secreted *in vitro* and target specific antigen epitopes. Screening and obtaining high-purity monoclonal antibodies is of great significance for scientific research, disease diagnosis, and treatment. Despite recent advancements in monoclonal antibody screening technologies, there remains a need for new and improved methods for enrichment and high-throughput screening of antigen-specific antibody-secreting cells.

### SUMMARY

The present disclosure provides, in part, a universal, high-throughput antibody discovery platform based on: (i) a negative-selection method for enriching B cells or plasma B cells without relying on plasma B cell surface biomarkers; and/or (ii) a microfluidic droplet-based functional screening system capable of identifying single cells secreting antigen-specific antibodies.

In some embodiments, the enrichment method employs a customizable cocktail of antibodies that target non-B lineage immune cells (*e.g.,* T cells, monocytes, dendritic cells, NK cells, IgM naïve B cells, and/or granulocytes), followed by magnetic bead depletion. It is believed that in some embodiments, the method does not require a positive biomarker such as CD138/CD38, and therefore it is applicable to species lacking reliable plasma cell markers, including rabbit, camelid, non-human primates, canine, bovine, ovine, and other mammals.

In some embodiments, enriched antibody-secreting cells are subsequently encapsulated into picoliter or nanoliter droplets together with (a) antigen-coated beads, (b) a fluorescent labeled antigen, and/or (c) antigen-expressing reporter cells, allowing for functional detection of secreted antibodies inside each droplet. In some embodiments, through multi-channel fluorescence detection-incorporating detection antibody signals, dead-cell exclusion dyes, and optional blocking-antibody controls-droplets containing antigen-specific antibody-secreting cells are isolated. The recovered cells can undergo RT-PCR and/or sequencing to obtain naturally paired antibody heavy and light chain sequences.

In some embodiments, this platform integrates cell enrichment, secretion-based specificity screening, single-cell isolation, and BCR sequence recovery into a single workflow, applicable to any species and any antigen class including soluble proteins, membrane proteins, conformational antigens, and complex multi-pass receptors.

In an aspect, the disclosure provides a method for enriching and identifying antigen-specific antibody-secreting cells, comprising:
(a) contacting a heterogeneous immune cell population with at least one depletion antibody that specifically binds non-B-lineage cells of the same species, wherein the at least one depletion antibody is coupled (*e.g*., conjugated) to a tag, optionally wherein the tag is biotin, fluorophore, a peptide tag, an affinity tag, a chemical label, or any combination thereof, thereby producing depletion-antibody-labeled cells;
(b) removing the depletion-antibody-labeled cells from the heterogeneous immune cell population by adding affinity beads capable of specifically binding the tag to the heterogeneous immune cell population, optionally wherein the affinity beads comprise anti-biotin beads, anti-fluorophore beads, anti-tag beads, streptavidin beads, avidin beads, nickel-nitrilotriacetic acid (Ni-NTA) beads, glutathione beads, or any combination thereof, thereby producing an enriched population of B cells or plasma B cells, optionally wherein at least 50% (e.g., at least 60%, 70%, 75%, 80%, 85%, 90%, or 95%) of the cells in the population are B cells or plasma B cells;
(c) co-encapsulating the enriched population of B cells or plasma B cells into microfluidic droplets together with (A):
   (i) antigen-coated beads; and/or
   (ii) antigen-expressing reporter cells; and

   with at least one detection antibody capable of binding immunoglobulin secreted by the enriched population of B cells or plasma B cells; or
   together with (B):
      (iii) an antigen (*e.g.,* a labeled (*e.g.,* fluorescently labeled) antigen) and immunoglobulin-capture (*e.g.,* IgG-capture) antibody-coated beads; and
   optionally, with at least one detection antibody capable of binding immunoglobulin secreted by the enriched population of B cells or plasma B cells, *e.g.,* to differentiate IgG and IgM;
(d) incubating the droplets to allow antibody secretion;
(e) detecting a localized signal (*e.g.,* fluorescence signal) indicative of antigen-specific antibody binding;
(f) sorting positive droplets;
(g) recovering antibody-secreting cells from the positive droplets; and
(h) optionally, amplifying nucleic acids encoding immunoglobulin heavy and/or light chains, or a fragment thereof, from the antibody-secreting cells.

In some embodiments, the tag is biotin, FITC, PE, APC, Cy5, HA-tag, FLAG-tag, His-tag, GST-tag, SNAP-tag, CLIP-tag, DIG, DNP, or a derivative thereof.

In some embodiments, the affinity beads are 0.1-10 µm magnetic particles. In some embodiments, the affinity beads are 0.2-5 µm, 0.5-2 µm, 0.1-5 µm, 0.1-2 µm, 0.1-1 µm, 0.1-0.5 µm, 0.1-0.2 µm, 5-10 µm, 2-10 µm, 1-10 µm, 0.5-10 µm, 0.2-10 µm, 0.5-1 µm, 0.5-1.5 µm, 1-2 µm, 2-3 µm, 3-4 µm, 4-5 µm, 5-6 µm, 6-7 µm, 7-8 µm, 8-9 µm, or 9-10 µm magnetic particles. In some embodiments, the affinity beads are coated with an anti-tag antibody, an affinity ligand, a peptide binder, a metal, a polymer, or any combination thereof.

In some embodiments, the at least one depletion antibody is chosen according to the species of origin and comprises an antibody recognizing one or more of: a T-cell marker, an NK-cell marker, a monocyte marker, a macrophage marker, a dendritic-cell marker, a granulocyte marker, or an IgM-positive naive B-cell marker. In some embodiments, the species is rabbit, rat, hamster, bovine, ovine, canine, feline, camelid, llama, alpaca, a non-human primate, or any combination thereof.

In some embodiments, the at least one depletion antibody comprises a plurality of depletion antibodies. In some embodiments, the plurality of depletion antibodies recognizes two or more (*e.g.,* three, four, five, six, or seven) of: a T-cell marker, an NK-cell marker, a monocyte marker, a macrophage marker, a dendritic-cell marker, a granulocyte marker, or an IgM-positive naïve B-cell marker. In some embodiments, the at least one detection antibody is labeled (*e.g.,* fluorescently labeled).

In some embodiments, the method further comprises treating (a) the enriched population of B cells or plasma B cells; (b) the antigen-coated beads and/or the antigen-expressing reporter cells; or (c) both (a) and (b), with a blocking antibody or a FcR-blocking reagent to reduce non-specific interactions, *e.g.,* prior to the co-encapsulating of step. In some embodiments, the blocking antibody or reagent is labeled (*e.g.,* fluorescently labeled).

In some embodiments, the detection further comprises using a viability dye. In some embodiments, the viability dye is NUCGREEN^{™}, NUCRED^{™}, EVAGREEN^{™}, a SYTOX^{™} dye, or an equivalent thereof. In some embodiments, droplets comprising dead cells are excluded.

In some embodiments, the antigen-coated beads comprise polystyrene, silica, agarose, dextran, a polymer, or a hydrogel. In some embodiments, the antigen-coated beads comprise magnetic beads having a diameter of 20 nm to 50 µm. In some embodiments, the antigen-coated beads comprise magnetic beads having a diameter of 50 nm to 20 µm, 100 nm to 10 µm, 200 nm to 5 µm, 500 nm to 2 µm, 20 nm to 20 µm, 20 nm to 10 µm, 20 nm to 5 µm, 20 nm to 2 µm, 20 nm to 1 µm, 20 nm to 500 nm, 20 nm to 200 nm, 20 nm to 100 nm, 20 nm to 50 nm, 20 µm to 50 µm, 10 µm to 50 µm, 5 µm to 50 µm, 2 µm to 50 µm, 1 µm to 50 µm, 500 nm to 50 µm, 200 nm to 50 µm, 100 nm to 50 µm, 50 nm to 50 µm, 50 nm to 100 nm, 100 nm to 200 nm, 200 nm to 500 nm, 500 nm to 1 µm, 1 µm to 2 µm, 2 µm to 5 µm, 5 µm to 10 µm, or 10 µm to 20 µm.

In some embodiments, the antigen-expressing reporter cells express a membrane protein, a multi-pass receptor, a G protein-coupled receptor (GPCR), an ion channel, a viral glycoprotein, or another conformational antigen.

In some embodiments, the antigen is labeled (*e.g.,* fluorescently labeled).

In some embodiments, at least 2% (*e.g.,* at least 5%, 10%, 15%, 20%, 25%, or 30%) of the encapsulated microfluidic droplets comprise one or two B cells or plasma B cells. In some embodiments, 15%-25% (*e.g.,* about 20%) of the encapsulated microfluidic droplets comprise one or two B cells or plasma B cells. In some embodiments, at least 2% (*e.g.,* at least 5%, 10%, 15%, 20%, 25%, or 30%) of the encapsulated microfluidic droplets comprise one B cell or plasma B cell. In some embodiments, 15%-25% (*e.g.,* about 20%) of the encapsulated microfluidic droplets comprise one B cell or plasma B cell.

In some embodiments, at least 75% (*e.g.,* at least 80%, 85%, 90%, 95%, or 99%) of the encapsulated microfluidic droplets comprise one or two antigen-expressing reporter cells. In some embodiments, 85%-95% (*e.g.,* about 90%) of the encapsulated microfluidic droplets comprise one or two antigen-expressing reporter cells. In some embodiments, at least 75% (*e.g.,* at least 80%, 85%, 90%, 95%, or 99%) of the encapsulated microfluidic droplets comprise one antigen-expressing reporter cell. In some embodiments, 85%-95% (*e.g.,* about 90%) of the encapsulated microfluidic droplets comprise one antigen-expressing reporter cell.

In some embodiments, at least 2% *(e.g.,* at least 5%, 10%, 15%, 20%, 25%, or 30%) of the encapsulated microfluidic droplets comprise one or two B cells or plasma B cells, and at least 75% (*e.g.,* at least 80%, 85%, 90%, 95%, or 99%) of the encapsulated microfluidic droplets comprise one or two antigen-expressing reporter cells. In some embodiments, 15%-25% (*e.g.,* about 20%) of the encapsulated microfluidic droplets comprise one or two B cells or plasma B cells, and 85%-95% (*e.g.,* about 90%) of the encapsulated microfluidic droplets comprise one or two antigen-expressing reporter cells. In some embodiments, at least 2% (*e.g.,* at least 5%, 10%, 15%, 20%, 25%, or 30%) of the encapsulated microfluidic droplets comprise one B cell or plasma B cell, and at least 75% (*e.g.,* at least 80%, 85%, 90%, 95%, or 99%) of the encapsulated microfluidic droplets comprise one antigen-expressing reporter cell. In some embodiments, 15%-25% (*e.g.,* about 20%) of the encapsulated microfluidic droplets comprise one B cell or plasma B cell, and 85%-95% (*e.g.,* about 90%) of the encapsulated microfluidic droplets comprise one antigen-expressing reporter cell.

In some embodiments, at least 2% (*e.g.,* at least 5%, 10%, 15%, 20%, or 25%) of the encapsulated microfluidic droplets comprise (a) one or two B cells or plasma B cells; and (b) one or two antigen-expressing reporter cells. In some embodiments, 10%-20% of the encapsulated microfluidic droplets comprise (a) one or two B cells or plasma B cells; and (b) one or two antigen-expressing reporter cells. In some embodiments, at least 2% (*e.g.,* at least 5%, 10%, 15%, 20%, or 25%) of the encapsulated microfluidic droplets comprise (a) one B cell or plasma B cell; and (b) one antigen-expressing reporter cell. In some embodiments, 10%-20% of the encapsulated microfluidic droplets comprise (a) one B cell or plasma B cell; and (b) one antigen-expressing reporter cell.

In some embodiments, a droplet is classified as positive when:
(a) the detection antibody generates a signal (*e.g.,* fluorescence signal) that exceeds a predetermined threshold; and
(b) the signal (*e.g.,* fluorescence signal) generated by dead cells is at background level; and
(c) optionally, the signal (*e.g.,* fluorescence signal) generated by the blocking antibody is at background level.

In some embodiments, the sorting is performed by dielectrophoresis, acoustics forces, pneumatic valves, mechanical deflection, optical sorting, or surface-energy-based sorting.

In some embodiments, nucleic acids encoding immunoglobulin variable heavy and/or light chains are amplified, for example, by RT-PCR. In some embodiments, nucleic acids encoding immunoglobulin variable heavy and/or light chains are sequenced, *e.g.,* after amplification. In some embodiments, the sequencing is performed using Sanger sequencing, Illumina sequencing, PacBio sequencing, Oxford Nanopore sequencing, or any combination thereof.

In another aspect, the disclosure provides a method for enriching B cells, comprising:
(a) contacting a heterogeneous immune cell population with at least one depletion antibody that specifically binds non-B-lineage cells of the same species, wherein the at least one depletion antibody is coupled (*e.g.,* conjugated) to a tag, optionally wherein the tag is biotin, fluorophore, a peptide tag, an affinity tag, a chemical label, or any combination thereof, thereby producing depletion-antibody-labeled cells; and
(b) removing the depletion-antibody-labeled cells from the heterogeneous immune cell population by adding affinity beads capable of specifically binding the tag to the heterogeneous immune cell population, optionally wherein the affinity beads comprise anti-biotin beads, anti-fluorophore beads, anti-tag beads, streptavidin beads, avidin beads, nickel-nitrilotriacetic acid (Ni-NTA) beads, glutathione beads, or any combination thereof, thereby producing an enriched population of B cells or plasma B cells, optionally wherein at least 50% (e.g., at least 60%, 70%, 75%, 80%, 85%, 90%, or 95%) of the cells in the population are B cells or plasma B cells.

In some embodiments, the tag is biotin, FITC, PE, APC, Cy5, HA-tag, FLAG-tag, His-tag, GST-tag, SNAP-tag, CLIP-tag, DIG, DNP, or a derivative thereof.

In some embodiments, the affinity beads are 0.1-10 µm magnetic particles. In some embodiments, the affinity beads are 0.2-5 µm, 0.5-2 µm, 0.1-5 µm, 0.1-2 µm, 0.1-1 µm, 0.1-0.5 µm, 0.1-0.2 µm, 5-10 µm, 2-10 µm, 1-10 µm, 0.5-10 µm, 0.2-10 µm, 0.5-1 µm, 0.5-1.5 µm, 1-2 µm, 2-3 µm, 3-4 µm, 4-5 µm, 5-6 µm, 6-7 µm, 7-8 µm, 8-9 µm, or 9-10 µm magnetic particles. In some embodiments, the affinity beads are coated with an anti-tag antibody, an affinity ligand, a peptide binder, a metal, a polymer, or any combination thereof.

In some embodiments, the at least one depletion antibody is chosen according to the species of origin and comprises an antibody recognizing one or more of: a T-cell marker, an NK-cell marker, a monocyte marker, a macrophage marker, a dendritic-cell marker, a granulocyte marker, or an IgM-positive naive B-cell marker. In some embodiments, the species is rabbit, rat, hamster, bovine, ovine, canine, feline, camelid, llama, alpaca, a non-human primate, or any combination thereof.

In some embodiments, the at least one depletion antibody comprises a plurality of depletion antibodies. In some embodiments, the plurality of depletion antibodies recognizes two or more (*e.g.,* three, four, five, six, or seven) of: a T-cell marker, an NK-cell marker, a monocyte marker, a macrophage marker, a dendritic-cell marker, a granulocyte marker, or an IgM-positive naïve B-cell marker. In some embodiments, the at least one detection antibody is labeled (e.g., fluorescently labeled).

In yet another aspect, the disclosure provides a method for identifying an antigen-specific antibody-secreting cell, the method comprising:
(a) providing microfluidic droplets co-encapsulated with a population of B cells (e.g., an enriched population of B cells, *e.g.,* as described herein) together with (A):
   (i) antigen-coated beads; and/or
   (ii) antigen-expressing reporter cells; and

   with at least one detection antibody capable of binding immunoglobulin secreted by the population of B cells; or
   together with (B):
      (iii) a labeled (*e.g.,* fluorescently labeled) antigen and immunoglobulin-capture antibody-coated beads; and
   optionally, with at least one detection antibody capable of binding immunoglobulin secreted by the population of B cells, *e.g.,* to differentiate IgG and IgM,
(b) incubating the droplets to allow antibody secretion;
(c) detecting droplets exhibiting a localized fluorescence signal indicative of antigen-specific antibody binding;
(d) sorting positive droplets; and
(e) recovering antibody-secreting cells from the positive droplets.

In some embodiments, (a) the population of B cells or plasma B cells; (b) the antigen-coated beads, the antigen-expressing reporter cells, and/or immunoglobulin-capture antibody-coated beads; or (c) both (a) and (b), have been treated with a blocking antibody or a FcR-blocking reagent to reduce non-specific interactions, *e.g*., prior to the co-encapsulating of step. In some embodiments, the method further comprises treating (a) the population of B cells or plasma B cells; (b) the antigen-coated beads, the antigen-expressing reporter cells, and/or immunoglobulin-capture antibody-coated beads; or (c) both (a) and (b), with a blocking antibody or a FcR-blocking reagent to reduce non-specific interactions, *e.g*., prior to the co-encapsulation step. In some embodiments, the blocking antibody or reagent is labeled (*e.g*., fluorescently labeled).

In some embodiments, the detection further comprises using a viability dye. In some embodiments, the viability dye is NUCGREEN^{™}, NUCRED^{™}, EVAGREEN^{™}, a SYTOX^{™} dye, or an equivalent thereof. In some embodiments, droplets comprising dead cells are excluded.

In some embodiments, the antigen-coated beads comprise polystyrene, silica, agarose, dextran, a polymer, or a hydrogel. In some embodiments, the antigen-coated beads comprise magnetic beads having a diameter of 20 nm to 50 µm. In some embodiments, the antigen-coated beads comprise magnetic beads having a diameter of 50 nm to 20 µm, 100 nm to 10 µm, 200 nm to 5 µm, 500 nm to 2 µm, 20 nm to 20 µm, 20 nm to 10 µm, 20 nm to 5 µm, 20 nm to 2 µm, 20 nm to 1 µm, 20 nm to 500 nm, 20 nm to 200 nm, 20 nm to 100 nm, 20 nm to 50 nm, 20 µm to 50 µm, 10 µm to 50 µm, 5 µm to 50 µm, 2 µm to 50 µm, 1 µm to 50 µm, 500 nm to 50 µm, 200 nm to 50 µm, 100 nm to 50 µm, 50 nm to 50 µm, 50 nm to 100 nm, 100 nm to 200 nm, 200 nm to 500 nm, 500 nm to 1 µm, 1 µm to 2 µm, 2 µm to 5 µm, 5 µm to 10 µm, or 10 µm to 20 µm.

In some embodiments, the antigen-expressing reporter cells express a membrane protein, a multi-pass receptor, a G protein-coupled receptor (GPCR), an ion channel, a viral glycoprotein, or another conformational antigen.

In some embodiments, the antigen is labeled (*e.g.,* fluorescently labeled).

In some embodiments, at least 2% *(e.g.,* at least 5%, 10%, 15%, 20%, 25%, or 30%) of the encapsulated microfluidic droplets comprise one or two B cells or plasma B cells. In some embodiments, 15%-25% (*e.g.,* about 20%) of the encapsulated microfluidic droplets comprise one or two B cells or plasma B cells. In some embodiments, at least 2% (*e.g.,* at least 5%, 10%, 15%, 20%, 25%, or 30%) of the encapsulated microfluidic droplets comprise one B cell or plasma B cell. In some embodiments, 15%-25% (*e.g.,* about 20%) of the encapsulated microfluidic droplets comprise one B cell or plasma B cell.

In some embodiments, at least 75% (*e.g.,* at least 80%, 85%, 90%, 95%, or 99%) of the encapsulated microfluidic droplets comprise one or two antigen-expressing reporter cells. In some embodiments, 85%-95% (*e.g.,* about 90%) of the encapsulated microfluidic droplets comprise one or two antigen-expressing reporter cells. In some embodiments, at least 75% (*e.g.,* at least 80%, 85%, 90%, 95%, or 99%) of the encapsulated microfluidic droplets comprise one antigen-expressing reporter cell. In some embodiments, 85%-95% (*e.g.,* about 90%) of the encapsulated microfluidic droplets comprise one antigen-expressing reporter cell.

In some embodiments, at least 2% (*e.g.,* at least 5%, 10%, 15%, 20%, 25%, or 30%) of the encapsulated microfluidic droplets comprise one or two B cells or plasma B cells, and at least 75% (*e.g.,* at least 80%, 85%, 90%, 95%, or 99%) of the encapsulated microfluidic droplets comprise one or two antigen-expressing reporter cells. In some embodiments, 15%-25% (*e.g.,* about 20%) of the encapsulated microfluidic droplets comprise one or two B cells or plasma B cells, and 85%-95% (*e.g.,* about 90%) of the encapsulated microfluidic droplets comprise one or two antigen-expressing reporter cells. In some embodiments, at least 2% (*e.g.,* at least 5%, 10%, 15%, 20%, 25%, or 30%) of the encapsulated microfluidic droplets comprise one B cell or plasma B cell, and at least 75% (*e.g.,* at least 80%, 85%, 90%, 95%, or 99%) of the encapsulated microfluidic droplets comprise one antigen-expressing reporter cell. In some embodiments, 15%-25% (*e.g.,* about 20%) of the encapsulated microfluidic droplets comprise one B cell or plasma B cell, and 85%-95% (*e.g.,* about 90%) of the encapsulated microfluidic droplets comprise one antigen-expressing reporter cell.

In some embodiments, at least 2% (*e.g.,* at least 5%, 10%, 15%, 20%, or 25%) of the encapsulated microfluidic droplets comprise (a) one or two B cells or plasma B cells; and (b) one or two antigen-expressing reporter cells. In some embodiments, 10%-20% of the encapsulated microfluidic droplets comprise (a) one or two B cells or plasma B cells; and (b) one or two antigen-expressing reporter cells. In some embodiments, at least 2% (*e.g.,* at least 5%, 10%, 15%, 20%, or 25%) of the encapsulated microfluidic droplets comprise (a) one B cell or plasma B cell; and (b) one antigen-expressing reporter cell. In some embodiments, 10%-20% of the encapsulated microfluidic droplets comprise (a) one B cell or plasma B cell; and (b) one antigen-expressing reporter cell.

In some embodiments, a droplet is classified as positive when:
(a) the detection antibody generates a signal (*e.g.,* fluorescence signal) that exceeds a predetermined threshold; and
(b) the signal (*e.g.,* fluorescence signal) generated by dead cells is at background level; and
(c) optionally, the signal (*e.g.,* fluorescence signal) generated by the blocking antibody is at background level.

In some embodiments, the sorting is performed by dielectrophoresis, acoustics forces, pneumatic valves, mechanical deflection, optical sorting, or surface-energy-based sorting.

In some embodiments, nucleic acids encoding immunoglobulin variable heavy and/or light chains are amplified, for example, by RT-PCR. In some embodiments, nucleic acids encoding immunoglobulin variable heavy and/or light chains are sequenced, *e.g.,* after amplification. In some embodiments, the sequencing is performed using Sanger sequencing, Illumina sequencing, PacBio sequencing, Oxford Nanopore sequencing, or any combination thereof.

In another aspect, the disclosure provides a method for enriching single B cells, comprising the following steps:
S1. incubating a single-cell suspension with a biotinylated specific antibody composition, such that antibodies in the composition specifically bind to non-target cells to be removed; and
S2. adding streptavidin-coated magnetic beads to the cell suspension from S1, and subjecting the mixture to magnetic separation to remove the non-target cells bound to the antibodies, thereby obtaining an enriched population of single B cells,
wherein said antibody composition comprises one or more antibodies selected from the group consisting of an anti-T lymphocyte antibody, an anti-CD4 antibody, an anti-CD8 antibody, an anti-CD11b antibody, an anti-IgM antibody, an anti-CD11c antibody, and an anti-CD123 antibody.

In some embodiments, in said cell suspension, the concentrations of the specific antibodies are: the anti-T lymphocyte antibody 1 µg/mL-30 µg/mL, the anti-CD4 monoclonal antibody 1 µg/mL-30 µg/mL, the anti-CD8 monoclonal antibody 1 µg/mm-30 µg/mL, the anti-CD11b monoclonal antibody 1 µg/mL-50 µg/mL, the anti-IgM monoclonal antibody 1 µg/mL-40 µg/mL, the anti-CD11c monoclonal antibody 1 µg/mL-30 µg/mL, and the anti-CD123 monoclonal antibody 1 µg/mm-30 µg/mL.

In some embodiments, in said cell suspension, the concentrations of the specific antibodies are: the anti-T lymphocyte antibody 5 µg/mL-30 µg/mL, the anti-CD4 monoclonal antibody 5 µg/mL-30 µg/mL, the anti-CD8 monoclonal antibody 5 µg/mm-30 µg/mL, the anti-CD11b monoclonal antibody 10 µg/mL-50 µg/mL, the anti-IgM monoclonal antibody 10 µg/mL-40 µg/mL, the anti-CD11c monoclonal antibody 5 µg/mL-30 µg/mL, and the anti-CD123 monoclonal antibody 5 µg/mL-30 µg/mL.

In some embodiments, the antibodies are specific for rabbit antigens. In some embodiments, the anti-CD4 monoclonal antibody is an anti-rabbit CD4 monoclonal antibody, the anti-CD8 monoclonal antibody is an anti-rabbit CD8 monoclonal antibody, the anti-CD11b monoclonal antibody is an anti-human or rabbit CD11b monoclonal antibody, the anti-IgM monoclonal antibody is an anti-rabbit IgM monoclonal antibody, the anti-CD11c monoclonal antibody is a mouse anti-human or rabbit CD11c monoclonal antibody, and the anti-CD123 monoclonal antibody is a mouse anti-human or rabbit CD123 monoclonal antibody.

In some embodiments, the antibodies are specific for camelid (*e.g.,* llama or alpaca) antigens, wherein the anti-CD4 monoclonal antibody is an anti-camelid CD4 monoclonal antibody, the anti-CD8 monoclonal antibody is an anti-camelid CD8 monoclonal antibody, the anti-CD11b monoclonal antibody is an anti-camelid CD11b monoclonal antibody, the anti-IgM monoclonal antibody is an anti-camelid IgM monoclonal antibody, the anti-CD11c monoclonal antibody is an anti-camelid CD11c monoclonal antibody, and the anti-CD123 monoclonal antibody is an anti-camelid CD123 monoclonal antibody.

In some embodiments, the single B cells are from a rabbit, rat, hamster, bovine, ovine, canine, feline, camelid, llama, alpaca, non-human primate, or any combination thereof. In some embodiments, the single-cell suspension is obtained from a rabbit, rat, hamster, bovine, ovine, canine, feline, camelid, llama, alpaca, non-human primate, or any combination thereof.

In some embodiments, said specific antibodies are a combination of at least 4 antibodies selected from the anti-T lymphocyte antibody, the anti-CD4 monoclonal antibody, the anti-CD8 monoclonal antibody, the anti-CD11b monoclonal antibody, the anti-IgM monoclonal antibody, the anti-CD11c monoclonal antibody, and the anti-CD123 monoclonal antibody.

In some embodiments, the tissue source of the single cells comprises spleen and peripheral blood.

In yet another aspect, the disclosure provides a microfluidic screening method for single B cells, comprising:
(a) obtaining an enriched single B cell suspension according to a method described herein;
(b) providing antigen-coated microbeads by coating the antigen protein onto the surface of microbeads;
(c) encapsulating the single B cell suspension and the antigen-coated microbeads into a plurality of nanoliter or picoliter droplets using a microfluidic device, wherein the cells and microbeads are suspended in a buffer containing a detection antibody carrying a first fluorescent label;
(d) incubating the droplets to allow the single B cells to secrete target specific antibodies, which form a complex with the antigen-coated microbeads and the detection antibody within the droplets;
(e) detecting multi-channel fluorescent signals from the incubated droplets and sorting target droplets based on a fluorescent signal value; and
(f) recovering target single B cells from the sorted droplets.

In some embodiments, said buffer further comprises a dead cell fluorescent dye, and the fluorescent signal generated by said first fluorescent label is different from the fluorescent signal generated by the dead cell fluorescent dye.

In some embodiments, the method further comprises performing reverse transcription and PCR amplification on the screened target single B cells, and obtaining the corresponding antibodies expressed by the B cells through sequencing and expression verification.

In some embodiments, the single B cells are from a rabbit, rat, hamster, bovine, ovine, canine, feline, camelid, llama, alpaca, non-human primate, or any combination thereof. In some embodiments, the single-cell suspension is obtained from a rabbit, rat, hamster, bovine, ovine, canine, feline, camelid, llama, alpaca, non-human primate, or any combination thereof.

In still another aspect, the disclosure provides a method for high-throughput screening monoclonal antibodies secreted by single plasma cells, characterized in that the method comprises:
(1) providing a plurality of nanoliter or picoliter droplets, each droplet comprising:
   a. no more than one antibody-secreting cell;
   b. microbeads coated with a target antigen, or at least one reporter cell expressing the target antigen on cell surface;
   c. a detection antibody and/or a dead cell fluorescent dye;
(2) incubating the plurality of droplets under suitable conditions to allow the antibody-secreting cell to secrete the target antibody, thereby obtaining incubated droplets;
(3) passing the incubated droplets individually through a droplet sorting chip to perform multi-fluorescent signal detection, and sorting and enriching target droplets based on the fluorescent signal values of the droplets,

wherein in step (1), the antibody-secreting cells are pre-incubated with a blocking antibody; and the reporter cell expressing the target antigen is pre-incubated with a blocking antibody, and
wherein said blocking antibody specifically binds to the target antibody; said detection antibody specifically binds to the target antibody; and said blocking antibody cannot be recognized or bound by said detection antibody; wherein the detection antibody carries a first fluorescent label, and the fluorescent signal produced by the first fluorescent label is distinct from the fluorescent signal produced by the dead cell fluorescent dye.

In some embodiments, the target droplets comprise: (i) droplets whose fluorescent signal value in the detection antibody fluorescent channel shows a statistically significant difference from the droplet background fluorescent value; and (ii) droplets whose fluorescent signal value in the dead cell dye fluorescent channel shows no statistically significant difference from the droplet background fluorescent value.

In some embodiments, the target droplets comprise: 1) droplets whose fluorescent signal value in the detection antibody fluorescent channel is at least 1.2 times the droplet background fluorescent value; and 2) droplets whose fluorescent signal value in the dead cell dye fluorescent channel is less than or equal to 1.1 times the droplet background fluorescent value.

In some embodiments, the blocking antibody carries a second fluorescent label, and the fluorescent signal produced by the second fluorescent label is distinct from the fluorescent signal produced by the first fluorescent label.

In some embodiments, the target droplets further comprise: (iii) droplets whose fluorescent signal value in the blocking antibody fluorescent channel shows no statistically significant difference from the droplet background fluorescent value.

In some embodiments, the target droplets further comprise: 3) droplets whose fluorescent signal value in the blocking antibody fluorescent channel shows no statistically significant difference from the droplet background fluorescent value.

In some embodiments, the statistically significant difference refers to a p-value < 0.05; and the no statistically significant difference refers to a p-value > 0.1.

In some embodiments, the preparation of the droplets comprises the following steps:
S1. preparing the antibody-secreting cells into a single-cell suspension, adding a blocking antibody for a blocking treatment, and washing the cells after the blocking treatment to remove excess blocking antibody, thereby obtaining a single-cell suspension of blocked antibody-secreting cells;
S2. preparing the reporter cells expressing the target antigen into a single-cell suspension, adding a blocking antibody for a blocking treatment, and washing the cells after the blocking treatment to remove excess blocking antibody, thereby obtaining a single-cell suspension of blocked reporter cells; or coating the target antigen on the microbeads to obtain antigen-coated microbeads;
S3. preparing a buffer solution containing the detection antibody and/or the dead cell fluorescent dye, and removing large particulate fluorescent substances from the buffer solution via filtration or high-speed centrifugation to obtain a filtered droplet assay buffer;
S4. resuspending the blocked antibody-secreting cells and the blocked reporter cells respectively in the filtered droplet assay buffer, and mixing the resuspended suspensions to generate a plurality of nanoliter or picoliter droplets; or, resuspending the blocked antibody-secreting cells and the antigen-coated microbeads respectively in the filtered droplet assay buffer, and mixing the resulting suspensions to generate a plurality of nanoliter or picoliter droplets.

In some embodiments, in step S3, the filter membrane used for filtration is a bacterial filter membrane of 0.45 µm or below; and the centrifugal force for high-speed centrifugation is at least 8000 × g.

In some embodiments, the blocking antibody and the detection antibody are each independently selected from at least one of a monoclonal antibody and a polyclonal antibody.

In some embodiments, the first fluorescent label comprises any one fluorescent group selected from the group consisting of Alexa Fluor 488, Alexa Fluor 647, PE, and BV421. In some embodiments, the second fluorescent label comprises any one fluorescent group selected from the group consisting of Alexa Fluor 488, Alexa Fluor 647, PE, and BV421. In some embodiments, the fluorescent signal produced by the second fluorescent label is distinct from the fluorescent signal produced by the first fluorescent label.

### BRIEF DESCRIPTION OF THE DRAWINGS

The accompanying figures illustrate typical embodiments and workflow steps.
**FIG. 1** is a schematic diagram illustrating the high-throughput screening of single plasma B cell antibodies, comprising plasma B cell enrichment, blocking of plasma B cells or reporter cells, droplet encapsulation and incubation, and the sorting and collection of positive droplets.
**FIG. 2** is a schematic diagram illustrating the workflow for plasma B cell enrichment from heterogeneous cell populations (*e.g.,* PBMCs, splenocytes, or other lymphoid cells), comprising: S1, mixing a biotinylated antibody cocktail with the cell mixture and incubating to allow the antibodies to bind to non-target cells; and S2, adding streptavidin-coated magnetic beads and performing magnetic separation to remove the unwanted cells bound by the antibodies, thereby obtaining enriched plasma B cells.
**FIGS. 3A-3C** illustrate three types of antigen presentation systems and their corresponding droplet assays, wherein: **FIG. 3A** shows antigen-coated beads; **FIG. 3B** shows fluorescently labeled antigens; and **FIG. 3C** shows antigens over-expressed on the surface of reporter cells. Modes A, B, and C are independent and may be combined in same droplet or separate assays.

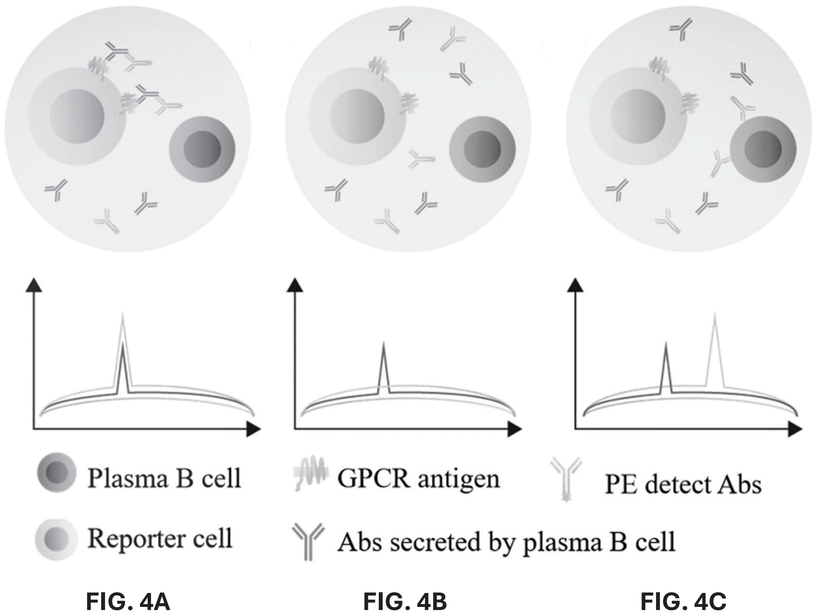

**FIGS. 4A-4C** illustrate the fluorescence signal patterns of a positive droplet (**FIG. 4A**), a negative droplet (**FIG. 4B**), and a false-positive droplet (**FIG. 4C**). Within each graph, the middle line indicates the fluorescence of the reporter cell stain, wherein a peak signifies the presence of a reporter cell within the droplet; the top line indicates the fluorescence of the detection antibody (PE), wherein a peak signifies antibody binding to cells (indicating specific binding in **FIG. 4A** or binding to non-target cells in **FIG. 4C**); and the bottom line indicates the fluorescence of the NUCGREEN^{™} dye, wherein a peak signifies the presence of a dead cell. The false-positive signals typically originate from non-specific binding of the secondary detection antibody to dead cells (which exhibit internal protein exposure due to cell membrane damage with high probability) or to living cells, or from specific binding of the detection antibody to memory B cells remaining in the enriched plasma B cell population.
**FIG. 5** illustrates the cell types and their respective proportions in samples enriched from rabbit PBMCs following the depletion of non-B lineage cells using various antibody cocktails in Examples 1-2, wherein the cell identities and percentages were determined via single-cell RNA sequencing and downstream analysis.
**FIG. 6** illustrates the cell types and their respective proportions in samples enriched from rabbit splenocytes following the depletion of non-B lineage cells using various antibody cocktails in Examples 3-7, wherein the cell identities and percentages were determined via single-cell RNA sequencing and downstream analysis
**FIGS. 7A-7B** present the ELISpot analysis of IgG-secreting plasma B cells enriched from rabbit splenocytes using different antibody cocktails (corresponding to Examples 3-7), wherein **FIG. 7A** depicts the results at a seeding density of 5000 cells per well, and **FIG. 7B** depicts the results at a seeding density of 1000 cells per well. The spot counts indicate the frequency of functional antibody-secreting cells, demonstrating the enrichment efficiency of the respective cocktails compared to the control.
**FIG. 8** illustrates the percentage of IgG-secreting cells in enriched samples from different groups, wherein plasma B cells were enriched from rabbit splenocytes using various antibody cocktails **FIG. 9** presents the ELISpot analysis of IgG-secreting plasma B cells enriched from Llama PBMC using antibody cocktails in Examples 9, wherein depicts the results at a seeding density of 10,000 cells per well. The spot counts indicate the frequency of functional antibody-secreting cells, demonstrating the enrichment efficiency of the respective cocktails compared to the control.
**FIG. 10** illustrates the percentage of IgG-secreting cells in enriched samples, wherein plasma B cells were enriched from Llama PBMC using antibody cocktails.
**FIG. 11** is a fluorescent image of microdroplets obtained in Example 10.
**FIG. 12** is a signal profile diagram of positive droplets identified in Example 10.
**FIG. 13** is a scatter plot displaying droplet signals and the results of positive droplet identification in Example 10.
**FIG. 14** provides the ELISA validation results of antibodies recovered from the screened positive droplets in Example 10.
**FIGS. 15A-15B** present microscopy images of droplets prepared using droplet assay buffer with filtration (**FIG. 15A**) and without filtration (**FIG. 15B**) in Example 11, wherein bright fluorescent spots are observed in the non-filtered group, while no bright spots are present in the filtered group.
**FIGS. 16A-16B** illustrate the analysis of droplets prepared from droplet assay buffer with filtration **(****FIG. 16A****)** or without filtration (**FIG. 16B**) by the droplet sorter in Example 11, showing that droplets exhibiting high fluorescence values in the PE channel (detection antibody) constitute approximately 1.55% of the population in the unfiltered group, compared to only 0.03% in the filtered group using the same criteria.
**FIGS. 17A-17D** present bright-field and fluorescence microscopy images of droplets prepared using samples with blocking of the plasma B cells and reporter cells (**FIGS. 17A-17B****,** respectively) and without blocking of the plasma B cells and reporter cells (**FIGS. 17C-17D****,** respectively), wherein cells binding to the PE detection antibody are observed in the non-blocking group in Example 12, whereas no bright cells are present in the blocking group.

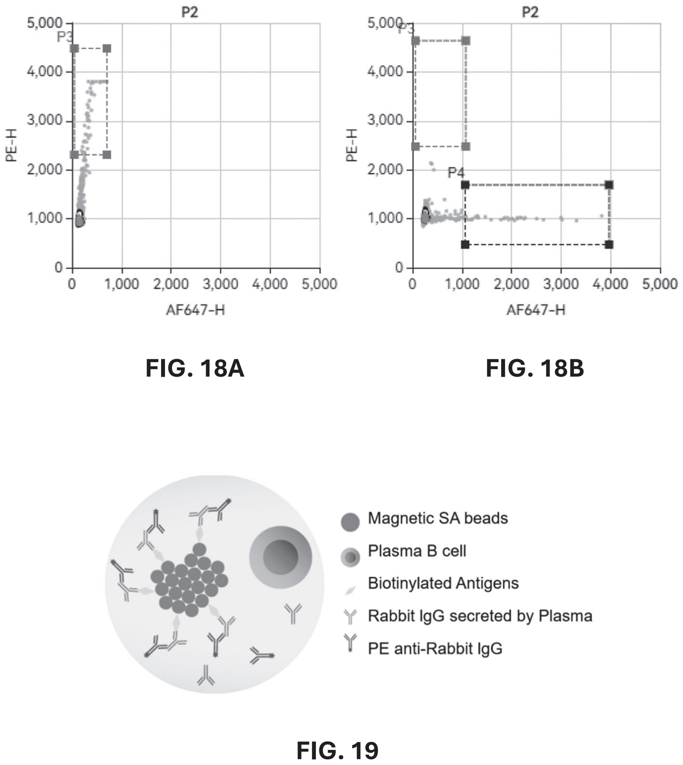

**FIGS. 18A-18B** illustrate the analysis of droplets prepared using samples with blocking of the plasma B cells and reporter cells (**FIG. 18A**) and without blocking of the plasma B cells and reporter cells (**FIG. 18B**) in Example 12, showing that droplets exhibiting high fluorescence values in the PE channel (detection antibody) constitute a significantly larger population in the non-blocking group compared to the blocking group using the same criteria. Additionally, droplets exhibiting high fluorescence values in the AF647 channel in the blocking group demonstrate the non-specific binding that occurred during the blocking step.
**FIG. 19** is a schematic diagram illustrating the structural components of a picoliter droplet during microfluidic screening.
**FIGS. 20A-20B** illustrate the signal scatter plots and positive droplet identification results of the droplets in the NUCGREEN^{™} channel (**FIG. 20A**) and the PE channel (**FIG. 20B**) in Example 13.
**FIG. 21** presents an agarose gel electrophoresis image of BCR products derived from positive droplets obtained in Example 13.
**FIG. 22** presents the ELISA results of antigen-antibody binding assays performed on purified antibodies from 20 randomly selected products obtained in Example 13.
**FIGS. 23A-23B** present bright-field images (**FIG. 23A**) and fluorescence images (**FIG. 23B**) of the droplets in Example 14, displaying the detection antibody (PE channel) and the blocking antibody (AF647 channel).
**FIGS. 24A-24B** illustrate the signal scatter plots and positive droplet identification results of the droplets in the NUCGREEN^{™} channel (**FIG. 24A**) and the PE -AF647 dual channels (**FIG. 24B**) in Example 14.
**FIG. 25** presents the FACS verification results for the screening of positive droplets against membrane protein antigen targets in Example 14.

### DETAILED DESCRIPTION

The following detailed description illustrates various embodiments of the disclosure. These embodiments are exemplary and non-limiting. Unless otherwise defined, all technical terms have the meaning commonly understood by those skilled in immunology, molecular biology, and microfluidics.

The disclosure provides, in part, a workflow that integrates: (a) species-adaptable negative selection of B cells or plasma B cells; (b) microfluidic droplet encapsulation with antigen-presenting elements; (c) fluorescence-based functional screening of secreted antibodies; (d) droplet sorting and single-cell recovery; and (e) amplification and sequencing of paired heavy and light chain genes.

Currently, various technologies are available for screening monoclonal antibodies, with flow cytometric sorting (FACS) and hybridoma fusion being the most widely applied. However, these methods have notable limitations. FACS is primarily used to obtain surface antibodies from memory B cells; it is difficult to utilize this method for screening secreting antibodies or antibodies targeting membrane surface antigens. Hybridoma technology is applicable only to limited species and is characterized by low throughput, long development cycles, and heavy workloads. Furthermore, it is difficult to cope with samples containing a low rate of positive cells.

Techniques based on fluorescence image recognition and optical manipulation have also been employed to screen secreting monoclonal antibodies. However, these solutions suffer from high usage costs, relatively low throughput, and strict requirements for plasma cell sample quality, which have prevented their large-scale adoption. Microfluidic platforms have recently emerged to allow single cells to be isolated with their secreted antibodies in droplets. Despite their potential, existing approaches still suffer from technical bottlenecks, including: non-specific aggregation on beads leading to false positives; dead cell debris generating background fluorescence, which obscures true signals; auto-fluorescent particulates leading to detection noise; difficulty in screening membrane protein antigens, often due to complex assay requirements; and inconsistent sensitivity in species with low antibody secretion rates or low B-cell frequencies.

While plasma cells in model organisms like humans and mice can be enriched using positive markers (e.g., CD138, CD38), this approach faces limitations. First, validated plasma cell biomarkers are unavailable for many species, such as rabbit, camelid, non-human primates, canine, bovine, ovine, and other mammals, making positive selection unfeasible. Second, in unenriched samples, the frequency of antigen-specific plasma cells is exceedingly low (often<0.01%), rendering reliable identification inefficient. Third, contaminating populations in primary tissues-particularly macrophages in PBMCs or splenocytes-are prone to forming cell clumps, which can lead to severe clogging of microfluidic channels. Finally, these non-target cells frequently exhibit non-specific absorption of fluorescent detection antibodies, generating negative or false-positive signals that obscure true hits. Therefore, plasma cell enrichment may be a necessary pre-treatment step.

The present disclosure addresses these challenges, in part, by integrating upstream sample enrichment with downstream high-fidelity screening to substantially increase the overall accuracy of antibody discovery. For example, the methods described herein can overcome one or more or all these limitations by providing: (a) a species-independent negative-selection method targeting non-B-lineage cells without any requirement for positive plasma cell markers; (b) a fully integrated droplet microfluidic workflow enabling direct functional screening of antibody secretion; (c) compatibility with soluble antigens and membrane protein antigens (via antigen-coated beads, fluorescent labeled antigen, or antigen-expressing cells); (d) high sensitivity through blocking antibodies, dead-cell exclusion, and background-reduction strategies; and (e) direct retrieval of paired heavy/light chain sequences from each selected cell.

In an aspect, the present disclosure provides a universal, high-throughput platform for discovering antigen-specific antibodies from single B cells or plasma B cells, applicable across multiple mammalian species including those lacking validated plasma cell markers (e.g., rabbit, camelid, non-human primates, canine, bovine, ovine, and other mammals). In some embodiments, the disclosure integrates three major technical modules as follows.

### I. Species-adaptable negative-selection enrichment of plasma B cells

A heterogeneous immune cell population (*e.g*., PBMC, splenocytes, lymph node cells, bone marrow cells) can be subjected to a species-adaptable negative selection process. The process involves, for example:
1) selecting one or more antibodies that specifically bind non-B lineage cells of the species under study (*e.g*., CD4⁺ and CD8⁺ T cells, monocytes/macrophages, dendritic cells, NK cells, granulocytes, IgM⁺ naive B cells). These depletion antibodies are conjugated with a detectable or capturable tag, such as, biotin, fluorophores (PE, FITC, APC, etc.), peptide tags (HA, FLAG, Myc, etc.), His-tag, GST-tag, or other affinity ligands; 2) removing the labeled cells using magnetic beads capable of specifically binding the tag, including: anti-biotin beads, streptavidin/avidin beads, anti-fluorophore beads, anti-biotin beads, or any affinity bead matched to the antibody's label.

In some embodiments, this strategy does not rely on species-specific positive markers such as CD138. Instead, the operator chooses an antibody panel appropriate for the species sample, offering a universal, adaptable purification workflow.

### II. Microfluidic droplet-based functional screening of antibody secretion

The enriched plasma cells or antibody expressing cells can be co-encapsulated into water-in-oil microdroplets together with:
(A) antigen-coated beads (magnetic or non-magnetic, 20 nm-50 µm);
(B) fluorescent labeled antigen with IgG-capture antibody coated bead; and/or
(C) antigen-expressing reporter cells, such as CHO cells stably expressing membrane proteins or multi-pass receptors.

Within each droplet, antibodies secreted by a single cell can bind to the bead- or cell-presented antigen. The bound antibodies can be detected by fluorescent secondary antibodies, producing a local fluorescence cluster within the droplet.

To increase specificity and reduce false positives, the disclosure can include:

### 1) Blocking antibodies

Before encapsulation, both the antibody secreting cells and the antigen-presenting cells/beads are treated with non-labeled or differently labeled blocking antibodies (*e.g.*, anti-FcR blockers, anti-species-matched IgG secondary antibody). This reduces nonspecific interactions or specific binding of secondary antibody to memory B left in enriched samples.

### 2) Dead-cell exclusion dyes

A nucleic acid dye such as NUCGREEN^{™}, NUCRED^{™}, EVAGREEN^{™}, or other viability markers can be added to distinguish droplets containing dead cells or debris, eliminating a major source of false positive fluorescence coming from unspecific binding of fluorescent-label antigen or secondary antibody to these dead cells or debris.

### 3) Background-reduction steps

Buffers and reagents can be filtered (0.22-0.45µm) or centrifuged (≥ 8,000 g) to remove fluorescent aggregates.

### 4) Multichannel fluorescence gating

Positive droplets can be defined by high signal in the detection antibody channel, low or background signal in the dead-cell channel, and optionally, low or background in the blocking-antibody channel. This multi-parameter strategy yields high specificity even when antigen-specific cells represent <0.01% of the population.

### III. Recovery, molecular amplification, and sequencing of natural antibody gene pairs

Cells from sorted droplets can be lysed, and their immunoglobulin transcripts can be reverse transcribed. Heavy chain and light chain variable regions can be amplified using species-appropriate primer pools or universal primers. Resulting amplicons can be sequenced using, for example, Sanger sequencing, Illumina short-read sequencing, PacBio or Oxford Nanopore long-read sequencing, or any equivalent platform. Naturally paired VH-VL sequences are thereby preserved.

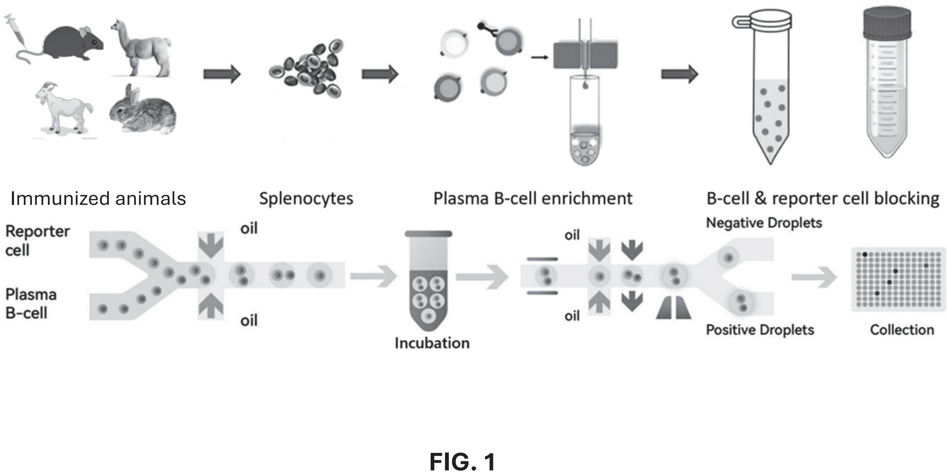

The present disclosure provides a species-independent platform that is particularly advantageous for species lacking stable plasma cell surface markers, such as rabbits. By utilizing a negative-selection strategy, the method avoids the reliance on positive-selection markers that can alter cell physiology or bias the secretory population. Furthermore, the platform is compatible with a broad spectrum of antigen classes, including soluble antigens, multimeric proteins, and membrane proteins such as GPCRs and ion channels. A high signal-to-noise ratio is achieved through the integration of blocking antibodies, dead-cell exclusion dyes, and background-reduction protocols. Unlike phage or yeast display technologies, the present disclosure advantageously preserves the naturally paired heavy and light chain sequences. Additionally, the integrated workflow allows for the completion of selection and clone retrieval within 1 to 2 days, thereby facilitating rapid antibody discovery. An exemplary workflow is illustrated in **FIG. 1****.**

### Definitions

As used herein, the articles "a" and "an" refer to one or to more than one (*e.g.,* to at least one) of the grammatical object of the article.

As used herein, the term "or" means, and is used interchangeably with, the term "and/or", unless context clearly indicates otherwise.

As used herein, the term "about" and "approximately" generally mean an acceptable degree of error for the quantity measured given the nature or precision of the measurements. Exemplary degrees of error are within 20 percent (%), typically, within 10%, and more typically, within 5% of a given value or range of values.

As used herein, the term "B cell" includes a naïve B cell, a memory B cell, a plasmablast, a plasma B cell, or any immunoglobulin-secreting cell.

As used herein, the term "plasma B cell" refers to a terminally differentiated, antibody-secreting B lineage cell. In species such as rabbit, validated surface markers analogous to human CD138 may not be available, and negative-selection enrichment may be used.

As used herein, the term "non-B-lineage cell" refers to an immune cell that is not B lineage, including T cell, NK cell, monocyte, macrophage, dendritic cell, granulocyte, and in some embodiments IgM⁺ naïve B cell.

As used herein, the term "species-adaptable negative-selection antibody" refers to an antibody chosen based on the species of origin (*e.g*., rabbit, camelid, non-human primates, canine, bovine, ovine, and other mammals) to bind a non-B cell and allow its removal.

As used herein, the term "tag" refers to any epitope, ligand, chemical group, or label coupled (*e.g.,* conjugated) to a depletion antibody (*e.g.,* biotin, PE, FITC, HA, FLAG, His-tag).

As used herein, the term "affinity bead" refers to a bead capable of binding a tag, including but not limited to, streptavidin/avidin bead, anti-biotin bead, anti-fluorophore bead, anti-tag bead, or a custom affinity bead.

As used herein, the term "droplet" refers to a water-in-oil microdroplet of picoliter-to-nanoliter volume formed via a microfluidic method.

As used herein, the term "detection antibody" refers to an antibody capable of binding to a secreted immunoglobulin, typically an anti-IgG Fc, anti-IgG(H+L), or anti-kappa/lambda light chain, and generating a detectable signal (*e.g.,* a fluorescent signal). In some embodiments, the detection antibody is an antibody chemically linked to a fluorescent dye, also known sometimes herein as a fluorescent antibody.

### Species-Adaptable Negative-Selection Enrichment

### Overview

Different mammalian species exhibit different immune marker profiles. Many species lack robust plasma cell markers (*e.g.,* rabbit, camelid, non-human primates, canine, bovine, ovine, and other mammals), and positive-selection strategies may alter cell physiology.

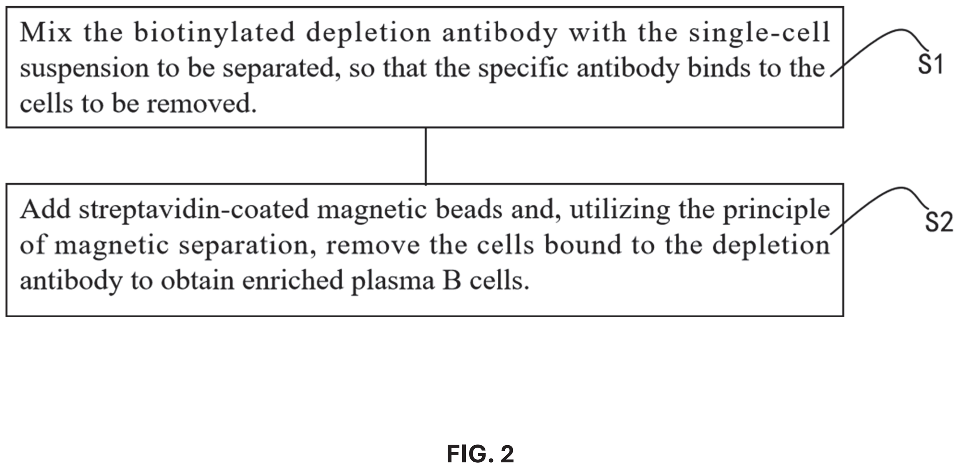

The disclosure provides, in part, a universal, species-adaptable negative-selection approach in which (**FIG. 2**) a panel of antibodies that recognize non-B cells is selected per species. These antibodies are tagged (*e.g.,* biotin/fluorophore/peptide tag). Tagged cells are removed by affinity magnetic beads matched to the tag. This process yields enriched populations of B cells or plasma B cells without relying on species-specific positive markers.

### Antibody tagging

The depletion antibodies may be commercially supplied pre-biotinylated antibodies, commercially supplied fluorophore-conjugated antibodies, or in-house labeled antibodies, using, for example, NHS-biotin chemistry, NHS-fluorophore labeling, maleimide-biotin, or peptide-tag chemical conjugation. The degree of labeling may range from 2-10 biotin molecules per antibody, or 1-5 fluorophore molecules per antibodies. The depletion antibodies may be whole-body IgG, F(ab)₂ IgG, F(ab) IgG, or nanobodies. Biotinylated antibodies are preferred in some embodiments.

### Affinity magnetic beads compatible with multiple tags

The beads are capable of binding the antibody tag, such as anti-biotin magnetic beads, streptavidin/avidin magnetic beads, anti-PE magnetic beads, anti-FITC magnetic beads, anti-HA magnetic beads, anti-FLAG magnetic beads, Ni-NTA magnetic beads (for His-tagged antibodies), glutathione magnetic beads (for GST-tagged antibodies). Beads may be 0.02-10 µm in diameter.

The anti-biotin/streptavidin magnetic beads are preferred in some embodiments.

### Species-specific depletion antibody panels

The species-depletion panel can be a combination of antibodies targeting one or more (*e.g.,* two, three, four, five, or all) of T cells, monocytes, DCs, macrophage, IgM⁺ naïve B cells, and IgD⁺ B cells. In some embodiments, the species-depletion panel is a combination (*e.g.,* two, three, four, five, or all) of antibodies selected from the group consisting of an anti-T lymphocyte antibody, an anti-CD4 antibody, an anti-CD8 antibody, an anti-CD11b antibody, an anti-IgM antibody, an anti-CD11c antibody, an anti-IgD antibody, and an anti-CD123 antibody. These antibodies target T cells, monocytes, DCs, macrophage, IgM⁺ naïve B cells, and IgD⁺ B cells. The antibodies selected for enrichment typically do not exhibit cross-reactivity with the secondary detection antibodies utilized in subsequent detection steps. The selection of said enrichment antibodies should be tailored to the specific species of the sample, utilizing either species-specific antibodies or antibodies capable of cross-reacting with similar species. The antibodies constituting the panel may be independently selected from monoclonal antibodies or polyclonal antibodies, with monoclonal antibodies being preferred.

### Microfluidic Droplet Encapsulation

### Device design

In some embodiments of the disclosure, droplets are generated using a standard flow-focusing microfluidic device in which one or more aqueous inlets are used to introduce suspensions of antibody-secreting cells, antigen-presenting beads, reporter cells, and fluorescent secondary antibodies, while a separate inlet supplies an oil phase containing an appropriate surfactant, such as a fluorinated oil supplemented with a non-ionic EA-type surfactant. The device produces highly monodisperse droplets with volumes typically ranging from approximately 10 to 300 picoliters, corresponding to droplet diameters of about 10 to 100 micrometers. Cell loading follows Poisson statistics. For plasma B cells or antibody-secreting cells, the aqueous phase is introduced at a concentration that provides an effective Poisson parameter λ in the range of approximately 0.05 to 0.6, thereby ensuring that the vast majority of droplets contain zero or one secreting cell. In contrast, antigen-expressing reporter cells are introduced at a higher concentration corresponding to a Poisson parameter λ of approximately 0.8 to 2, which results in most droplets containing at least one reporter cell capable of presenting antigen for downstream antibody detection.

### Antigen Presentation Systems

The present disclosure supports multiple antigen-presentation strategies suitable for high-throughput screening of antibody-secreting B cells or plasma cells in microfluidic droplets.

In particular, three general antigen-presentation modes can be used. These modes may be employed individually or in any combinational format.

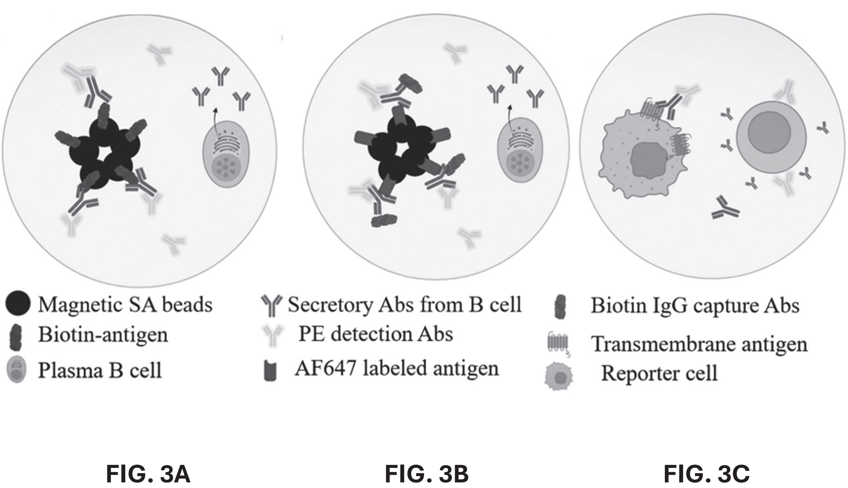

In certain embodiments, the antigen is immobilized on the surface of solid beads, as shown in **FIG. 3A**. The beads may be magnetic or non-magnetic and may have diameters ranging from 20 nm to 50 µm. Suitable bead materials include, but are not limited to, polystyrene, silica, agarose, dextran, sepharose, and proprietary polymeric or composite materials. A variety of antigen-attachment chemistries may be used, including, biotin-streptavidin or biotin-anti-biotin coupling, EDC/NHS-mediated amide formation, epoxy-based covalent attachment, maleimide-thiol conjugation, or any equivalent antibody- or ligand-binding surface. In this mode, droplets typically encapsulate at least one bead carrying surface-bound antigen, enabling rapid capture of secreted antibodies and fluorescent detection of antigen-antibody complexes.

In other embodiments, the antigen is present in soluble form within the droplet and is optionally fluorescently labeled (*e.g.,* FITC, Alexa Fluor dyes, PE, APC, Cy dyes), as shown in **FIG. 3B**. A secondary capture bead is co-encapsulated in the same droplet. The surface of the bead carries an antibody or affinity ligand capable of binding the Fc region or light chain of the secreted antibody. When a target-specific antibody is secreted by the B cell or plasma cell inside the droplet, the antibody first binds the soluble antigen and subsequently the antigen-antibody complex is captured onto the bead surface via the secondary antibody. This allows for local concentration of the fluorescent antigen signal onto the bead, greatly improving detection sensitivity and allowing background-free identification of positive droplets.

In another embodiment of the disclosure, the antigen is displayed on the surface of engineered reporter cells, which are co-encapsulated with the antibody-secreting cell inside the droplet as shown in **FIG. 3C****.** This mode is particularly advantageous for antigens requiring a native membrane environment, including, for example, membrane proteins, multi-pass receptors, ion channels, G-protein-coupled receptors (GPCRs), heteromeric receptor complexes, and antigens requiring specific lipid-raft or membrane microdomain localization. Suitable reporter cell types include, but are not limited to: CHO cells, HEK293 cells, K562 cells, Jurkat T cells, Ramos cells, and other engineered mammalian or hematopoietic cell lines. In typical implementations, droplets encapsulate at least one antigen-expressing reporter cell. Interaction between the secreted antibody and the cell-surface antigen is subsequently detected using fluorescent secondary antibodies or antigen-binding probes, thus allowing sorting and retrieval of positive droplets.

### Functional Antibody Detection Inside Droplets

A challenge in the design of microfluidic droplet assays for functional antibody detection is the suppression of false-positive signals to reliably distinguish them from true positive events. This discrimination is particularly important given that the frequency of true antigen-specific clones is typically exceedingly low, ranging from 0.1% to 0.01%, and in difficult cases, falling below 0.01%. As schematically illustrated in **FIGS. 4A-4C****,** false-positive droplets (**FIG. 4C**) frequently arise from three primary sources: (1) non-specific binding of fluorescent detection antibodies to dead cells or cellular debris, which often exhibit internal protein exposure due to compromise of membranes; (2) specific or non-specific binding of detection antibodies to surface markers on non-target living cells, such as memory B cells remaining in the enriched population; and (3) the formation of large fluorescent aggregates formed by the detection antibodies, labeled antigens or other fluorescent materials themselves. To overcome these limitations and ensure that the isolated droplets contain only viable, antigen-specific antibody-secreting plasma cells, in some embodiments the present disclosure integrates one or more (*e.g.,* all) of the following specific noise-reduction strategies.

### Secondary antibodies for detection

In certain embodiments of the disclosure, antibody detection within the droplet is achieved using a fluorophore-labeled secondary antibody directed against IgG, Igκ, Igλ, or the Fc region of the secreted immunoglobulin. Suitable fluorescent labels include, but are not limited to, PE, FITC, APC, and members of the Alexa Fluor dye family, although any functionally equivalent fluorophore may be employed. The secondary antibodies may be provided as full-length antibodies, F(ab) fragments, F(ab')₂ fragments, or nanobody formats, depending on the desired binding specificity and steric accessibility within the confined droplet environment. These reagents are typically used at final concentrations ranging from approximately 1 to 50 µg/mL, ensuring sufficient signal intensity for fluorescence-based identification and sorting of positive droplets.

### Blocking antibodies

In certain embodiments of the disclosure, the antibody-secreting cells, the antigen-presenting beads, and/or the antigen-expressing reporter cells are pre-treated with a blocking reagent prior to droplet encapsulation in order to minimize nonspecific interactions or unwanted specific interaction. In some implementations, such blocking reagents include unlabeled IgG derived from the same species as the fluorescent detection antibody, Fc-receptor blocking antibodies, or surface-masking immunoglobulins applied at concentrations of approximately 0.1 to 2 mg/mL. In various aspects of the disclosure, the blocking step serves to saturate Fc receptors or other reactive surfaces on the cells and beads, thereby reducing the likelihood of unintended interactions between the detection reagents and the assay components.

In preferred embodiments, the blocking antibody is selected from the same immunoglobulin origin as the secondary antibody for detection, but is either unlabeled or labeled with a distinct fluorophore, allowing irrelevant epitopes to be masked without interfering with the intended detection signal. In certain embodiments, two or more blocking antibodies directed toward different regions of the immunoglobulin molecule, such as anti-FcR antibodies combined with anti-IgG(H+L) antibodies, are employed concurrently to achieve comprehensive masking of nonspecific binding sites. In some implementations, this multipoint blocking strategy substantially suppresses false-positive events arising from nonspecific binding of the fluorescent secondary detection antibodies, thereby enhancing the accuracy with which droplets containing genuine antigen-specific antibody secretors can be identified.

### Dyes for dead cell exclusion

In certain embodiments of the disclosure, a dye for dead cell exclusion, such as NUCGREEN^{™}, NUCRED^{™}, EVAGREEN^{™}, or a SYTOX^{™} dye, is introduced into the aqueous phase prior to encapsulation. These dyes penetrate cells with compromised membranes and generate a strong nuclear signal, thereby enabling reliable identification of droplets containing dead or damaged cells. In some implementations, one dye or a combination of dyes is used to maximize discrimination between viable and non-viable cells. Droplets exhibiting intense dead-cell fluorescence can be excluded during downstream sorting. In various aspects of the disclosure, the removal of dead cells is desired because membrane-damaged cells expose internal proteins, nucleic acids, and other components that contribute to nonspecific adsorption of fluorescent secondary antibodies, which increases background and may lead to false-positive events. Accordingly, the incorporation of dead-cell exclusion dyes substantially improves detection fidelity.

### Background reduction

In certain embodiments of the disclosure, background fluorescence is reduced by subjecting all aqueous buffers and detection reagents to filtration through 0.22 µm or 0.45 µm filters or by centrifugation at forces of at least 8,000 g. These processes remove auto-fluorescent particles, antibody aggregates, and fluorescent antigen complexes that may otherwise produce spurious signals within droplets. In some implementations, minimizing such contaminants is essential for reducing false-positive droplets and maintaining a high signal-to-background ratio during fluorescence-based sorting.

### Fluorescence cluster formation

In some embodiments of the disclosure, when a secreted antibody binds to an antigen-coated bead or an antigen-expressing reporter cell within the droplet, the fluorescent detection antibody accumulates at the site of antigen-antibody interaction. This results in the formation of a discrete and localized fluorescence cluster that is markedly brighter than the surrounding background. The presence of these spatially resolved fluorescence clusters serves as a reliable indicator of antigen-specific secretion and facilitates accurate selection of positive droplets during sorting.

### Droplet Detection and Sorting

In certain embodiments of the disclosure, droplets are directed through an optical interrogation region where they are illuminated by laser excitation, and fluorescence emission is measured using photomultiplier tubes, photodiodes, or equivalent detectors. Sorting is subsequently performed using any suitable physical displacement mechanism. In some implementations, dielectrophoretic deflection is employed to selectively divert positive droplets into a collection channel. In other implementations, acoustic actuation, mechanical micro-valves, or surface-tension-based switching elements are used to achieve directional sorting. Depending on the device design and inlet pressure stability, sorting rates typically range from approximately 10 to 1000 droplets per second.

### Recovery of Cells and Antibody Sequences

In various embodiments of the disclosure, droplets identified as positive are dispensed either into individual wells of a 96-well or 384-well plate or collected into a single tube. Droplet disruption may be achieved by adding 1H,1H,2H,2H-perfluoro-1-octanol, by surfactant destabilization, or by centrifugation, thereby releasing the cells into the aqueous phase. When collected into a single tube, the recovered cells may be subjected to single-cell RNA-sequencing workflows, including commercial platforms such as 10x Genomics or BD Rhapsody systems, to obtain full B-cell receptor (BCR) repertoires.

In embodiments involving well-based recovery, individual cells are lysed using a mild lysis buffer, and the immunoglobulin variable regions are amplified by RT-PCR. Primers may target species-specific IgG heavy chains, species-specific Igκ or Igλ light chains, or universal immunoglobulin leader and constant-region sequences. The resulting amplicons typically range from approximately 400 to 600 base pairs for variable-region fragments or 1 to 2 kilobases for full-length V(D)J sequences. Sequencing may be performed using Sanger sequencing, Illumina short-read sequencing, PacBio long-read sequencing, or Oxford Nanopore Technologies. Long-read platforms permit direct pairing of heavy- and light-chain sequences, providing complete antibody-repertoire information for downstream expression and characterization.

### Methods for Enriching Rabbit Single B Cells and Microfluidic Screening Methods

In an aspect, the present disclosure provides a method for the enrichment of rabbit single B cells and a microfluidic screening platform thereof. The enrichment protocol comprises: (a) incubating a rabbit single-cell suspension with a biotinylated antibody composition to specifically bind to non-target cell populations; (b) adding streptavidin-coated magnetic beads to the suspension to capture the biotinylated non-target cells ; and (c) performing magnetic separation to remove the non-target cells, thereby recovering an enriched population of rabbit B cells. Specifically, the antibody composition comprises antibodies targeting T lymphocytes, CD4, CD8, CD11b, IgM, CD11c, and CD123. This negative selection strategy achieves high-purity enrichment while maintaining cellular viability and physiological state, significantly increasing the proportion of antibody-secreting plasma cells for subsequent high-throughput microfluidic sorting.

Rabbit monoclonal antibodies may possess significant technical advantages over mouse antibodies. Firstly, the larger size of the rabbit spleen allows for the recovery of a greater number of B cells. Secondly, the rabbit immune system can recognize epitopes that are poorly immunogenic in mice, thereby providing broader antibody diversity. Furthermore, rabbit antibodies typically exhibit higher affinity and stability, with a relatively simpler structure that facilitates downstream antibody engineering and optimization.

Due to the limited availability of specific markers commercially available for rabbit B cells, current isolation methods primarily rely on traditional Fluorescence-Activated Cell Sorting (FACS). However, this approach is constrained by sorting throughput and varying degrees of physical damage to B cells, leading to a low efficiency in obtaining functional antibody sequences. Although existing methods disclose enrichment protocols, they suffer from issues such as insufficient cell purity and significant loss of cell viability.

There are currently three major routes for rabbit monoclonal antibody development: 1) hybridoma technology, which is hindered by the instability of rabbit myeloma cell lines and low fusion efficiency; 2) phage display, which results in the loss of natural heavy- and light-chain pairing during library construction, often necessitating time-consuming in vitro affinity maturation; and 3) single B cell sequencing or in vitro culture, which faces challenges such as poor stability of activation protocols and loss of diversity. While some methods utilize droplet microfluidics to sort CHO cells expressing rabbit antibodies, there remains a lack of efficient platforms for the direct sorting of primary rabbit plasma B cells.

The present disclosure aims, in part, to overcome the drawbacks of low enrichment purity, significant damage from flow sorting, and low efficiency in single-cell screening associated with certain existing technologies. Without wishing to be bound by theory, it is believed that in some embodiments it provides a high-viability method for enriching rabbit single B cells and an efficient microfluidic sorting platform.

To solve the above problems, the disclosure provides at least the following technical solutions.

In one aspect, the disclosure provides a method for enriching rabbit single B cells, comprising the following steps:
S1. incubating a rabbit single-cell suspension with a composition comprising a biotinylated antibody, such that the antibody specifically binds to non-target cells to provide labeled cells;
S2. adding streptavidin-coated magnetic beads and employing magnetic separation to remove labeled cells, thereby recovering an enriched population of rabbit single B cells,
wherein said antibody composition comprises one or more antibodies selected from the group consisting of: anti-T lymphocyte, anti-CD4, anti-CD8, anti-CD11b, anti-IgM, anti-CD11c, and anti-CD123 antibodies.

A further technical solution is that, in said cell suspension, the concentrations of the one or more specific antibodies are: anti-T lymphocyte antibody 1 µg/mL-30 µg/mL, anti-CD4 monoclonal antibody 1 µg/mL-30 µg/mL, anti-CD8 monoclonal antibody 1 µg/mL-30 µg/mL, anti-CD11b monoclonal antibody 1 µg/mL-50 µg/mL, anti-IgM monoclonal antibody 1 µg/mL-40 µg/mL, anti-CD11c monoclonal antibody 1 µg/mL-30 µg/mL, and/or anti-CD123 monoclonal antibody 1 µg/mL-30 µg/mL.

A further technical solution is that, in said cell suspension, the concentrations of the one or more specific antibodies are: anti-T lymphocyte antibody 5 µg/mL-30 µg/mL, anti-CD4 monoclonal antibody 5 µg/mL-30 µg/mL, anti-CD8 monoclonal antibody 5 µg/mL-30 µg/mL, anti-CD11b monoclonal antibody 10 µg/mL-50 µg/mL, anti-IgM monoclonal antibody 10 µg/mL-40 µg/mL, anti-CD11c monoclonal antibody 5 µg/mL-30 µg/mL, and/or anti-CD123 monoclonal antibody 5 µg/mL-30 µg/mL.

A further technical solution is that the tissue source of said rabbit single cells comprises rabbit spleen and/or rabbit peripheral blood.

A further technical solution is that said anti-CD4 monoclonal antibody is a rabbit anti-CD4 monoclonal antibody, said anti-CD8 monoclonal antibody is a rabbit anti-CD8 monoclonal antibody, said anti-CD11b monoclonal antibody is a human anti-CD11b monoclonal antibody, said anti-IgM monoclonal antibody is a rabbit anti-IgM monoclonal antibody, said anti-CD11c is a mouse anti-human CD11c monoclonal antibody, and said anti-CD123 monoclonal antibody is a mouse anti-human CD123 monoclonal antibody.

A further technical solution is that said one or more specific antibodies are a combination of at least 4 antibodies selected from an anti-T lymphocyte antibody, an anti-CD4 monoclonal antibody, an anti-CD8 monoclonal antibody, an anti-CD11b monoclonal antibody, an anti-IgM monoclonal antibody, an anti-CD11c monoclonal antibody, and an anti-CD123 monoclonal antibody.

A further technical solution is that said specific antibodies are a combination of at least 5 antibodies selected from an anti-T lymphocyte antibody, an anti-CD4 monoclonal antibody, an anti-CD8 monoclonal antibody, an anti-CD11b monoclonal antibody, an anti-IgM monoclonal antibody, an anti-CD11c monoclonal antibody, and an anti-CD123 monoclonal antibody.

A further technical solution is that said specific antibodies are a combination of at least 6 antibodies selected from an anti-T lymphocyte antibody, an anti-CD4 monoclonal antibody, an anti-CD8 monoclonal antibody, an anti-CD11b monoclonal antibody, an anti-IgM monoclonal antibody, an anti-CD11c monoclonal antibody, and an anti-CD123 monoclonal antibody.

A further technical solution is that said specific antibodies are a combination of all antibodies selected from an anti-T lymphocyte antibody, an anti-CD4 monoclonal antibody, an anti-CD8 monoclonal antibody, an anti-CD11 monoclonal antibody, an anti-IgM monoclonal antibody, an anti-CD11c monoclonal antibody, and an anti-CD123 monoclonal antibody.

In another aspect, the disclosure also provides a microfluidic screening method for rabbit single B cells, comprising the following steps:
obtaining an enriched rabbit single B cell suspension according to the rabbit single B cell enrichment method described in any of the above solutions;
coating a target antigen onto the surface of microbeads to obtain antigen-coated microbeads;
preparing a buffer containing a detection antibody, resuspending the rabbit single B cell suspension and the antigen-coated microbeads respectively in said buffer, incubating the resuspended rabbit single B cell suspension and the antigen-coated microbeads, and preparing into multiple nanoliter or picoliter droplets;
incubating said droplets under suitable conditions to allow rabbit single B cells to secrete target antibodies, to obtain incubated droplets; and
passing the incubated droplets individually through a droplet sorting chip, performing multi-channel fluorescent signal detection, sorting and enriching target droplets based on the fluorescent signal value of the droplets, to obtain target rabbit single B cells,
wherein said detection antibody carries a first fluorescent label.

A further technical solution is that said first fluorescent label comprises any one fluorescent group selected from Alexa Fluor 488, Alexa Fluor 647, PE, or BV421.

Further, in some embodiments, said buffer comprises a dead cell fluorescent dye *(e.g.,* NUCGREEN^{™}, NUCRED^{™}, or EVAGREEN^{™}) that specifically binds only to dead cells, with excitation/emission spectra distinct from the first fluorescent label and showing no non-specific binding to live cells or proteins.

A further technical solution is that, in said buffer, the final concentration of said detection antibody can be any concentration between 2 nM and 400 nM, preferably 2-200 nM, more preferably 10-80 nM. The final concentration of said dead cell fluorescent dye can be any concentration between 5 nM and 5 µM, preferably 50 nM-1 µM.

A further technical solution is that it further comprises performing reverse transcription and PCR amplification on the screened target rabbit single B cells, and obtaining the corresponding antibodies expressed by the rabbit B cells through sequencing and expression verification.

Without wishing to be bound by theory, it is believed that compared with the existing methods, the technical effects that can be achieved by the present disclosure include, for example, the following.

The rabbit single B cell enrichment method provided by the disclosure uses a combination of multiple specific antibodies to capture non-B cells in the system, without affecting the quantity and quality of B cells in the system, achieving the effect of rapidly enriching high-purity rabbit B cells from rabbit single cells, greatly increasing the proportion of secretory plasma B cells. The enrichment process of the disclosure does not cause damage to B cells, improves the purity of rabbit plasma B cells, and the enriched rabbit plasma B cells can be directly used for downstream microfluidic screening of rabbit plasma B antibodies, effectively improving the downstream screening efficiency.

The microfluidic screening method for rabbit single B cells provided by the disclosure uses the aforementioned enriched rabbit single B cells for screening, can perform high-throughput rapid sorting of target microdroplets, has the advantages of high accuracy and no damage to cells, and can shorten the work that takes months with traditional methods such as hybridoma screening to be completed within 1 day. Such method is applicable to plasma cells from species other than rabbit (*e.g.,* a species described herein).

### Additional aspects and embodiments

In another aspect, the present disclosure provides a method for enriching rabbit single B cells, comprising the following steps:
S1. incubating a rabbit single-cell suspension with a biotinylated specific antibody composition, such that the antibodies specifically bind to non-target cells; and
S2. adding streptavidin-coated magnetic beads to the cell suspension obtained in S1, and employing magnetic separation to remove the biotin-labeled non-target cells, thereby recovering an enriched population of untouched rabbit single B cells,
wherein said specific antibodies targeting non-target cell surface markers comprise a combination of: an anti-T lymphocyte antibody, an anti-CD4 monoclonal antibody, an anti-CD8 monoclonal antibody, an anti-CD11b monoclonal antibody, and an anti-IgM monoclonal antibody.

In some embodiments, in said cell suspension, the concentrations of the specific antibodies are: the anti-T lymphocyte antibody 1 µg/mL-30 µg/mL, the anti-CD4 monoclonal antibody 1 µg/mL-30 µg/mL, the anti-CD8 monoclonal antibody 1 µg/mL-30 µg/mL, the anti-CD11b monoclonal antibody 1 µg/mL-50 µg/mL, the IgM monoclonal antibody 1 µg/mL-40 µg/mL, the anti-CD11c monoclonal antibody 1 µg/mL-30 µg/mL, and the anti-CD123 monoclonal antibody 1 µg/mL-30 µg/mL.

In preferred embodiments, in said cell suspension, the concentrations of the specific antibodies are: the anti-T lymphocyte antibody 5 µg/mL-30 µg/mL, the anti-CD4 monoclonal antibody 5 µg/mL-30 µg/mL, the anti-CD8 monoclonal antibody 5 µg/mL-30 µg/mL, the anti-CD11b monoclonal antibody 10 µg/mL-50 µg/mL, the anti-IgM monoclonal antibody 10 µg/mL-40 µg/mL, the anti-CD11c monoclonal antibody 5 µg/mL-30 µg/mL, and the anti-CD123 monoclonal antibody 5 µg/mL-30 µg/mL.

In more preferred embodiments, in said cell suspension, the concentrations of the specific antibodies are: the anti-T lymphocyte antibody 10 µg/mL-30 µg/mL, the anti-CD4 monoclonal antibody 10 µg/mL-30 µg/mL, the anti-CD8 monoclonal antibody 10 µg/mL-30 µg/mL, the anti-CD11b monoclonal antibody 10 µg/mL-50 µg/mL, the anti-IgM monoclonal antibody 10 µg/mL-40 µg/mL, the anti-CD11c monoclonal antibody 10 µg/mL-30 µg/mL, and the anti-CD123 monoclonal antibody 10 µg/mL-30 µg/mL.

In some embodiments, the rabbit single B cell enrichment method provided by the disclosure utilizes a negative selection strategy to capture non-B cells without affecting the physiological quality or viability of the target B cells. This allows for a rapid recovery of high-purity rabbit B cells and significantly increases the frequency of antibody-secreting plasma B cells available for downstream analysis.

In some embodiments of the rabbit single B cell enrichment method provided by the disclosure, the tissue source of said rabbit single cells comprises rabbit spleen and rabbit peripheral blood, which can be memory B cells and/or plasma B cells.

In some embodiments, the anti-CD4, anti-CD8, and anti-IgM antibodies are anti-rabbit monoclonal antibodies; the anti-CD11b is a human-specific antibody, and the anti-CD11c and anti-CD123 are mouse anti-human antibodies. In some embodiments, these heterologous antibodies are cross-reactive with corresponding rabbit antigens, facilitating the comprehensive depletion of rabbit monocytes, T lymphocytes, and dendritic cells (DCs).

In another aspect, the disclosure further provides a microfluidic screening method for rabbit single B cells, comprising:
obtaining an enriched rabbit single B cell suspension according to the enrichment method described above;
coating a target antigen protein onto the surface of microbeads to prepare antigen-coated microbeads;
preparing a buffer containing a detection antibody, resuspending the B cells and antigen-coated microbeads in said buffer, and encapsulating the mixture into multiple nanoliter or picoliter droplets using a microfluidic device;
incubating the droplets under suitable conditions to allow B cells to secrete target antibodies, which form fluorescent immune-complexes within the droplets;
passing the incubated droplets individually through a sorting chip for fluorescence signal detection, and recovering target droplets based on a predefined signal threshold (*e.g*., Target Signal+ / Dead Cell Signal-).

In some embodiments, said first fluorescent label comprises any one fluorescent group selected from Alexa Fluor 488, Alexa Fluor 647, PE, or BV421.

In some embodiments, said buffer further comprises a dead cell fluorescent dye, and the fluorescent signal generated by said first fluorescent label is different from the fluorescent signal generated by the dead cell fluorescent dye.

In some embodiments, said dead cell fluorescent dye is a dye such as NUCGREEN^{™}, NUCRED^{™}, or EVAGREEN^{™} that specifically binds only to dead cells and has excitation and emission spectral information. It is understandable that said dead cell fluorescent dye does not non-specifically bind to live cells, microbeads, or proteins.

In some embodiments, in said buffer, the final concentration of said detection antibody can be any concentration between 2 nM and 400 nM, preferably 2-200 nM, more preferably 10-80 nM. The final concentration of said dead cell fluorescent dye can be any concentration between 5 nM and 5 µM, preferably 50 nM-1 µM.

In some embodiments, the method further comprises performing reverse transcription and PCR amplification on the obtained target rabbit single B cells, and obtaining rabbit antibody-expressing B cells through sequencing and expression verification.

It is understood that in some embodiments the disclosure uses microfluidic sorting technology to screen for antigen-specific plasma B cells from the enriched rabbit single B cells, which can complete screening and BCR amplification and pairing within 2 days, with the advantages of high throughput, high sensitivity, and high screening accuracy.

The present disclosure in some embodiments integrates negative selection enrichment with droplet microfluidics to screen antigen-specific plasma B cells, enabling the entire workflow-from cell isolation to BCR pairing-to be completed within 2 days. This approach offers high throughput and maintains the native cognate pairing of heavy and light chains. It can be applied in the development of antibody drugs and diagnostic antibodies to obtain high-affinity antibodies for subsequent clinical applications.

### Methods for High-Throughput Screening of Monoclonal Plasma Cell Secreted Antibodies

In another aspect, the disclosure provides a method for high-throughput screening of monoclonal antibodies secreted by single plasma cell, relating to the field of antibody screening technology. The method utilizes microdroplets as reaction vessels and employs large particulate substrates (e.g., microbeads or reporter cells) carrying target antigens to mediate the generation of aggregated strong fluorescent signals from positive secreted antibodies and detection antibodies, thereby achieving high-sensitivity quantitative detection. To minimize non-specific binding, the screening method uses pre-incubated cell samples and reporter cell samples with blocking antibodies. Additionally, dead cell fluorescent dyes are utilized for real-time labeling within the droplet system to exclude false-positive signals generated by autofluorescence or non-specific adsorption of dead cells, thereby increasing the success rate of subsequent cell culture and BCR amplification. This method effectively eliminates the primary non-target signals within the system, significantly enhances the true positive rate of screening products, and allows for the identification of target antibodies from samples with extremely low positive frequencies.

Monoclonal secreted antibodies are produced by a single B-cell clone, capable of continuous secretion *in vitro* and targeting specific antigenic epitopes. To screen and obtain monoclonal antibodies recognize different epitopes is of great importance for basic scientific research, clinical diagnosis, and therapeutic development.

Currently, several technologies are employed for monoclonal antibody screening, with flow cytometric cell sorting and hybridoma fusion being the most widely utilized. However, flow cytometry is primarily used to isolate surface antibodies from memory B cells and faces significant challenges when screening for secreted antibodies or antibodies targeting cell surface antigens. Hybridoma fusion is restricted to specific species and is further limited by low throughput, protracted development cycles, intensive labor requirements, and poor performance with low-positive-rate samples.

Alternative technologies utilizing fluorescent image recognition combined with optical manipulation have emerged for screening monoclonal secreted antibodies. Nevertheless, these solutions are hindered by high operational costs, relatively low throughput, and stringent requirements for plasma cell sample quality, preventing their widespread adoption.

While high-throughput screening methods based on microfluidic technology have been developed, these systems are often susceptible to various false-positive signals. Such interference leads to instability during the screening process and limits both signal sensitivity and the positive binding rate.

Consequently, there is an urgent technical need for a comprehensive, high-accuracy, and high-throughput screening method specifically optimized for monoclonal secreted antibodies.

To address the aforementioned deficiencies in the existing methods, the present disclosure provides a comprehensive, high-accuracy, and high-throughput screening method specifically designed for monoclonal antibodies secreted by single plasma cells.

To achieve this objective, the present disclosure provides the following technical solutions.

In an aspect, the present disclosure provides a method for high-throughput screening of monoclonal plasma cell-secreted antibodies, comprising the following steps:
(1) providing a plurality of nanoliter or picoliter droplets, wherein each droplet contains:
   (a) no more than one antibody-secreting cell;
   (b) microbeads coated with a target antigen, or at least one reporter cell expressing the target antigen on cell surface;
   (c) a detection antibody and/or a dead cell fluorescent dye;
(2) incubating the plurality of droplets under suitable conditions to allow the antibody-secreting cell to secrete the target antibody, thereby obtaining incubated droplets;
(3) passing the incubated droplets individually through a droplet sorting chip for multi-fluorescent signal detection, and subsequently sorting and enriching target droplets based on their fluorescent signal values.

In some embodiments, in step (1), both the antibody-secreting cell and the reporter cell expressing the target antigen are pre-incubated with a blocking antibody; the blocking antibody and the detection antibody are each capable of specifically binding to the target antibody; however, the blocking antibody is characterized in that it is not recognized or bound by the detection antibody.

Furthermore, in some embodiments, the detection antibody carries a first fluorescent label, which generates a fluorescent signal distinct from that produced by the dead cell fluorescent dye.

In a further technical aspect, the incubation in step (2) is performed at a temperature of 20-37°C for a duration of 0.5-8 hours.

Sorting and enrichment involve identifying target droplets and applying a dielectrophoretic force (or alternative methods) to direct them toward a target channel near the electrode side. The target droplets, containing cells capable of secreting antigen-specific antibodies, are collected as a fluid stream at the end of the target channel. The isolated cells may then be utilized for downstream applications, including cell culture, hybridoma preparation, B-cell receptor (BCR) sequence capture and sequencing, re-cloning, antibody production, and affinity characterization.

In certain embodiments, target droplets are identified and sorted based on the following fluorescent signal characteristics: (i) droplets exhibiting a statistically significant difference in fluorescent signal intensity within the detection antibody channel compared to the droplet background fluorescence; and (ii) droplets exhibiting no statistically significant difference in fluorescent signal intensity within the dead cell dye channel compared to the droplet background fluorescence.

Alternatively, the criteria for target droplets may be defined by specific signal-to-background ratios: 1) fluorescent signal intensity in the detection antibody channel is at least 1.2 times the droplet background fluorescence value; and 2) fluorescent signal intensity in the dead cell dye channel is less than or equal to 1.1 times the droplet background fluorescence value.

Further technical aspects are as follows: the blocking antibody may carry a second fluorescent label, producing a fluorescent signal that is distinct from the signal produced by the first fluorescent label (on the detection antibody).

The blocking antibody used in the system may comprise an unlabeled antibody, an antibody carrying the second fluorescent label, or a combination thereof.

When utilizing a labeled blocking antibody, target droplets must satisfy an additional requirement to ensure successful depletion of non-specific signals: (iii) droplets exhibiting no statistically significant difference in fluorescent signal intensity within the blocking antibody fluorescent channel compared to the droplet background fluorescence; or the target droplets further comprise: 3) droplets whose fluorescent signal value in the blocking antibody fluorescent channel shows no statistically significant difference from the droplet background fluorescent value.

Further technical aspects are as follows: the statistically significant difference refers to a p-value < 0.05; and the no statistically significant difference refers to a p-value > 0.1.

In some embodiments, the preparation of the droplets for high-throughput screening comprises the following sequential steps:
S1. preparing the antibody-secreting cells into a single-cell suspension, adding a blocking antibody for a blocking treatment, and washing the cells after the blocking treatment to remove excess blocking antibody, thereby obtaining a single-cell suspension of blocked antibody-secreting cells;
S2. preparing the reporter cells expressing the target antigen into a single-cell suspension, adding a blocking antibody for a blocking treatment, and washing the cells after the blocking treatment to remove excess blocking antibody, thereby obtaining a single-cell suspension of blocked reporter cells; or, coating the target antigen on the microbeads to obtain antigen-coated microbeads;
S3. preparing a buffer solution containing the detection antibody and/or the dead cell fluorescent dye, and removing large particulate fluorescent substances from the buffer solution via filtration or high-speed centrifugation to obtain a filtered droplet assay buffer;
S4. resuspending the blocked antibody-secreting cells and the blocked reporter cells respectively in the filtered droplet assay buffer, and mixing the resuspended suspensions to generate a plurality of nanoliter or picoliter droplets; or resuspending the blocked antibody-secreting cells and the antigen-coated microbeads respectively in the filtered droplet assay buffer, and mixing the resulting suspensions to generate a plurality of nanoliter or picoliter droplets.

Further technical aspects are as follows: In steps S1 and S2, the blocking treatment temperature is 0-37°C the blocking time can be from 30 seconds to 12 hours. Preferably, the blocking treatment temperature is 2-25°C; the blocking time can be from 5 to 20 minutes. More preferably, the blocking treatment temperature is 2-8°C; the blocking time can be from 5 to 10 minutes.

Further technical aspects are as follows: In step S3, the filter membrane used for filtration is a bacterial filter membrane of 0.45 µm or below; and the centrifugal force for high-speed centrifugation is at least 8000 × *g*.

Further technical aspects are as follows: antibody-secreting cells belong to the B-cell lineage, including plasma cells, hybridoma cells, or other secreting cell types. These may be derived from human peripheral blood mononuclear cells (PBMC) or other species and tissues.

Further technical aspects are as follows: blocking and detection antibodies are independently selected from monoclonal or polyclonal antibodies, such as goat anti-human IgG-Fc fragment antibody, non-human anti-human IgG-Fc fragment antibody, non-human anti-human IgG (heavy chain + light chain) antibody, or immunoglobulin antibody.

In the droplet assay buffer preparation in step S3, the final concentration of the detection antibody can be any concentration between 2 nM and 400 nM, preferably 2-200 nM, more preferably 10-80 nM.

During blocking treatment, the final concentration of the blocking antibody in the system can be any concentration between 2 nM and 10 µM, preferably 2 nM - 1 µM, more preferably 10-80 nM.

The detection antibody is characterized in that it specifically binds to the heavy chain Fc fragment of the target antibody, or alternatively, to any epitope of the complete target antibody comprising the heavy chain and/or light chain.

Similarly, the blocking antibody specifically binds to the heavy chain Fc fragment of the target antibody, or to any fragment of the complete target antibody comprising the heavy chain and/or light chain.

The methodology of the present disclosure is applicable to a wide range of antibody structures beyond native forms. This includes, but is not limited to, engineered antibodies with incomplete structures, single-chain antibodies, chimeric antibodies from different species, or antibodies containing specific deletions or mutations.

The blocking antibody and the detection antibody may be derived from the same species source or from different species sources.

The first fluorescent label comprises any one fluorescent group selected from the group consisting of Alexa Fluor 488, Alexa Fluor 647, PE, and BV421. The second fluorescent label comprises any one fluorescent group selected from the group consisting of Alexa Fluor 488, Alexa Fluor 647, PE, and BV421. The fluorescent signal generated by the second fluorescent label is distinct from that produced by the first fluorescent label to ensure accurate multi-channel sorting.

Dead cell fluorescent dyes, such as NUCGREEN^{™}, NUCRED^{™}, or EVAGREEN^{™}, are employed to specifically bind to dead cells based on their unique excitation and emission spectra. These dyes are characterized by their lack of non-specific binding to live cells, microbeads, or proteins.

Within the droplet assay buffer solution obtained in step S3, the final concentration of the dead cell fluorescent dye ranges between 5 nM and 5 µM, with a preferred concentration range of 50 nM to 1 µM.

The substrate microbeads utilized in the system include both magnetic and non-magnetic microbeads.

The microbead particle size is defined such that its maximum dimension (if non-spherical) is smaller than the droplet diameter. For example, the microbead particle size may range from 20 nm to 50 µm.

Compared with the existing methods, the technical effects achievable by the present disclosure include the following.

The present disclosure provides a method for high-throughput screening monoclonal antibodies secreted by single plasma cells. The method utilizes microdroplets as individual reaction containers and employs large particulate substrates, such as microbeads or reporter cells expressing target antigens. These substrates mediate the generation of aggregated, strong fluorescent signals from positive secreted antibodies and detection antibodies, enabling high-sensitivity quantitative detection. By pre-incubating cell and reporter cell samples with blocking antibodies, the method masks memory cell surface antibodies, FcR receptors, and other binding sites. This significantly reduces non-specific binding of detection antibodies and lowers background signal strength. Further, dead cell fluorescent dyes provide real-time labeling of cells that are dead before encapsulation or die during incubation. Utilizing negative selection methods allows for the exclusion of false-positive signals caused by dead cell autofluorescence or non-specific adsorption. This exclusion improves the success rates of subsequent downstream processes, specifically cell culture and BCR amplification.

Furthermore, in some embodiments, blocking antibodies and labels used during pre-incubation can also label impurities or non-target cells, allowing them to be excluded via negative selection to improve the true positive rate.

Furthermore, in some embodiments, prior to droplet preparation, membrane filtration or high-speed centrifugation is employed to remove large particulate fluorescent proteins or dyes from the system. This effectively eliminates false-positive signals originating from these substances.

In summary, the disclosure creates a high-sensitivity system that effectively identifies monoclonal secreted antibodies with high affinity while strictly excluding non-target signals, resulting in extremely high positive proportions and reproducibility. The method offers a simpler process, lower costs, and shorter turnaround times compared to existing technologies, providing significant improvements in efficiency and economy. Additionally, it is applicable to plasma cells from multiple species with no strict requirements for sample purity. It supports various antigen types, including soluble proteins, membrane surface antigens, small molecule peptides, and modified antigens. It is particularly suitable for genetically engineered samples and difficult samples characterized by low cell counts or extremely low positive rates.

### EXAMPLES

### Example 1. Plasma B Cell Enriched from PBMCs (Isolation P1)

This example/embodiment provides a method for enriching plasma B cells from rabbit PBMCs, comprising the following steps: S1. mixing biotinylated depletion antibodies with a suspension of rabbit PBMCs to be separated, allowing said depletion antibodies to bind to cells to be removed to obtain a cell suspension; S2. adding streptavidin-coated magnetic beads to the cell suspension obtained in S1, and utilizing the principle of magnetic separation to remove cells bound to said specific antibodies, thereby obtaining enriched rabbit plasma B cells.

The rabbit plasma B cells enriched in this embodiment were stained with a fluorescent viability dye, and live/dead counting was performed using a fluorescent cell counter. Statistical analysis showed that the proportion of viable cells exceeded 90%.

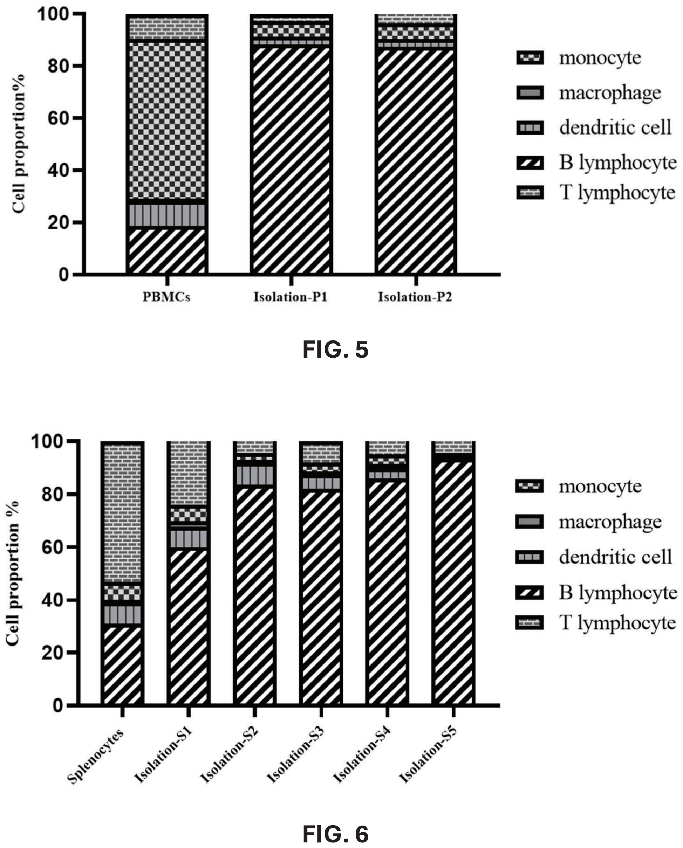

The depletion antibodies and their concentrations in this embodiment were: T lymphocyte antibody 8 µg/mL, CD4 monoclonal antibody 6 µg/mL, CD8 monoclonal antibody 6 µg/mL, CD11b monoclonal antibody 12 µg/mL, and IgM monoclonal antibody 10 µg/mL. As shown in **FIG. 5****,** the proportion of plasma B cells after enrichment increased by 4.7 times compared to the cells before enrichment.

### Example 2. Plasma B Cell Enriched from PBMCs (Isolation P2)

This example/embodiment provides a method for enriching plasma B cells from rabbit PBMCs, comprising the following steps: S1. mixing biotinylated depletion antibodies with a suspension of rabbit PBMCs to be separated, allowing said specific antibodies to bind to cells to be removed to obtain a cell suspension; S2. adding streptavidin-coated magnetic beads to the cell suspension obtained in S1, and utilizing the principle of magnetic separation to remove cells bound to said specific antibodies, thereby obtaining enriched rabbit plasma B cells.

The rabbit plasma B cells enriched in this embodiment were stained with a fluorescent viability dye, and live/dead counting was performed using a fluorescent cell counter. Statistical analysis showed that the proportion of viable cells exceeded 90%.

The depletion antibodies and their concentrations in this embodiment were: T lymphocyte antibody 8 µg/mL, CD4 monoclonal antibody 10 µg/mL, CD8 monoclonal antibody 10 µg/mL, CD11b monoclonal antibody 30 µg/mL, and IgM monoclonal antibody 10 µg/mL. As shown in **FIG. 5****,** the proportion of plasma B cells after enrichment increased by 4.6 times compared to the cells before enrichment.

### Example 3. Plasma B Cell Enriched from Splenocytes (Isolation S1)

This example/embodiment provides a method for enriching rabbit plasma B cells from rabbit splenocytes, comprising the following steps: S1. mixing biotinylated depletion antibodies with a suspension of rabbit splenocytes to be separated, allowing said depletion antibodies to bind to cells to be removed to obtain a cell suspension; S2. adding streptavidin-coated magnetic beads to the cell suspension obtained in and utilizing the principle of magnetic separation to remove cells bound to said specific antibodies, thereby obtaining enriched rabbit plasma B cells.

The depletion antibodies and their concentrations in this embodiment were: T lymphocyte antibody 10 µg/mL, CD4 monoclonal antibody 5 µg/mL, CD8 monoclonal antibody 5 µg/mL, CD11b monoclonal antibody 10 µg/mL, and IgM monoclonal antibody 10 µg/mL. As shown in **FIG. 6****,** relative to the cells before enrichment, the proportion of B cells after enrichment increased by 1.94 times, and the proportion of T cells decreased by 55%.

### Example 4. Plasma B Cell Enriched from Splenocytes (Isolation S2)

This example/embodiment provides a method for enriching plasma B cells from rabbit splenocytes, comprising the following steps: S1. mixing biotinylated depletion antibodies with a suspension of rabbit spleen cells to be separated, allowing said depletion antibodies to bind to cells to be removed to obtain a cell suspension; S2. adding streptavidin-coated magnetic beads to the cell suspension obtained in and utilizing the principle of magnetic separation to remove cells bound to said specific antibodies, thereby obtaining enriched rabbit single B cells.

The depletion antibodies and their concentrations in this embodiment were: T lymphocyte antibody 20 µg/mL, CD4 monoclonal antibody 10 µg/mL, CD8 monoclonal antibody 10 µg/mL, CD11b monoclonal antibody 10 µg/mL, and IgM monoclonal antibody 10 µg/mL. As shown in **FIG. 6****,** relative to the cells before enrichment, the proportion of B cells after enrichment increased by 2.7 times, and the proportion of T cells decreased by 90%.

### Example 5. Plasma B Cell Enriched from Splenocytes (Isolation S3)

This example/embodiment provides a method for enriching plasma B cells from rabbit splenocytes, comprising the following steps: S1. Mixing biotinylated depletion antibodies with a suspension of rabbit splenocytes to be separated, allowing said depletion antibodies to bind to cells to be removed to obtain a cell suspension; S2. adding streptavidin-coated magnetic beads to the cell suspension obtained in and utilizing the principle of magnetic separation to remove cells bound to said specific antibodies, thereby obtaining enriched rabbit plasma B cells.

The depletion antibodies and their concentrations in this embodiment were: T lymphocyte antibody 20 µg/mL, CD4 monoclonal antibody 10 µg/mL, CD8 monoclonal antibody 10 µg/mL, CD11b monoclonal antibody 10 µg/mL, IgM monoclonal antibody 10 µg/mL, and CD123 monoclonal antibody 5 µg/mL. As shown in **FIG. 6****,** relative to the cells before enrichment, the proportion of plasma B cells after enrichment increased by 2.55 times, the proportion of T cells decreased by 85%, and the proportion of DCs decreased by 30%.

### Example 6. Plasma B Cell Enriched from Splenocytes (Isolation S4)

This example/embodiment provides a method for enriching plasma B cells from rabbit splenocytes, comprising the following steps: S1. Mixing biotinylated depletion antibodies with a suspension of rabbit spleen cells to be separated, allowing said depletion antibodies to bind to cells to be removed to obtain a cell suspension; S2. Adding streptavidin-coated magnetic beads to the cell suspension obtained in and utilizing the principle of magnetic separation to remove cells bound to said specific antibodies, thereby obtaining enriched rabbit plasma B cells.

The depletion antibodies and their concentrations in this embodiment were: T lymphocyte antibody 20 µg/mL, CD4 monoclonal antibody 10 µg/mL, CD8 monoclonal antibody 10 µg/mL, CD11b monoclonal antibody 10 µg/mL, IgM monoclonal antibody 10 µg/mL, and CD11c monoclonal antibody 5 µg/mL. As shown in **FIG. 6****,** relative to the cells before enrichment, the proportion of rabbit plasma B cells after enrichment increased by 2.8 times, the proportion of T cells decreased by 85%, and the proportion of DCs decreased by 50%.

### Example 7. Plasma B Cell Enriched from Splenocytes (Isolation S5)

This example/embodiment provides a method for enriching plasma B cells from rabbit splenocytes, comprising the following steps: S1. Mixing biotinylated depletion antibodies with a suspension of rabbit spleen cells to be separated, allowing said depletion antibodies to bind to cells to be removed to obtain a cell suspension; S2. Adding streptavidin-coated magnetic beads to the cell suspension obtained in and utilizing the principle of magnetic separation to remove cells bound to said specific antibodies, thereby obtaining enriched rabbit single B cells.

The depletion antibodies and their concentrations in this embodiment were: T lymphocyte antibody 20 µg/mL, CD4 monoclonal antibody 15 µg/mL, CD8 monoclonal antibody 15 µg/mL, CD11b monoclonal antibody 10 µg/mL, IgM monoclonal antibody 10 µg/mL, CD11c monoclonal antibody 10 µg/mL, and CD123 monoclonal antibody 10 µg/mL. As shown in **FIG. 6****,** relative to the cells before enrichment, the proportion of plasma B cells after enrichment increased by 3 times, the proportion of T cells decreased by 91%, and the proportion of DCs decreased by 98%.

### Example 8. ELISpot Analysis of Plasma B Cells Enriched from Splenocytes

In this example/embodiment, the B cells enriched in splenocytes (Isolation S1-S5) were detected using the Enzyme-Linked ImmunoSpot (ELISpot) technique. The number of plasma B cells secreting IgG was used to represent the enriched plasma B cells. A rabbit splenocytes not subjected to the enrichment method of the present disclosure served as a control. The proportions of plasma B cells in each example were compared.

The ELISpot detection method was as follows: A 96-well PVDF membrane plate pre-treated with 35% alcohol was coated with 1 µg/mL goat anti-rabbit IgG (H+L) polyclonal antibody, 100 µL per well, and incubated at 4°C overnight; the next day, the plate was washed 5 times with 200 µL PBS, and blocked with RPMI 1640 medium containing 10% fetal bovine serum (FBS), 100 µL per well, incubated at 37°C for 1 hour; the plate was washed 5 times with 200 µL PBS. The first column was inoculated with 5000 cells per well, and the second column was inoculated with 1000 cells per well, 100 µL per well, and incubated in a cell culture incubator at 37°C overnight; the next day, the plate was washed 5 times with 200 µL PBS, and 100 µL of 1 µg/mL biotinylated goat anti-rabbit IgG Fc antibody was added and incubated at room temperature for 2 hours; the plate was washed 5 times with 200 µL PBS, and 100 µL of Streptavidin-ALP diluted 1:1000 was added and incubated at room temperature for 1 hour; the plate was washed 5 times with 200 µL PBS, and 100 µL of BCIP/NBT-plus chromogenic substrate was added and incubated at room temperature for 10 minutes; the reaction was terminated by washing 5 times with 200 µL PBS. After drying the PVDF membrane in an oven, the plate was photographed and analyzed using an upright microscope.

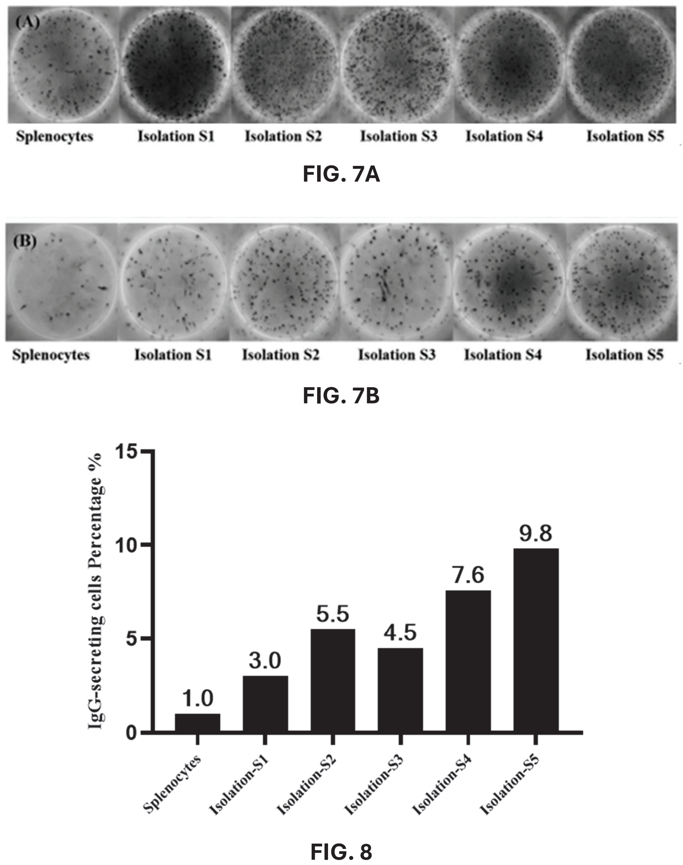

The results are shown in **FIGS. 7A-7B** and **FIG. 8**. After applying the enrichment method of the present disclosure to the rabbit splenocytes, the proportion of plasma B cells increased substantially. It can also be seen from the results of each example that the types and concentrations of depletion antibodies have a significant relationship with the proportion of the finally enriched plasma B cells.

### Example 9. Enrichment of Llama Plasma B Cells from PBMCs

This example/embodiment provides a method for enriching llama plasma B cells, comprising the following steps:
S1. mixing biotinylated specific antibodies with a suspension of llama PBMC cells to be separated, allowing said specific antibodies to bind to cells to be removed to obtain a cell suspension;
S2. adding streptavidin-coated magnetic beads to the cell suspension obtained in S1, and utilizing the principle of magnetic separation to remove cells bound to said specific antibodies, thereby obtaining enriched llama single B cells.

The llama B cells enriched in this embodiment were stained with a fluorescent viability dye, and live/dead counting was performed using a fluorescent cell counter. Statistical analysis showed that the proportion of viable cells exceeded 90%.

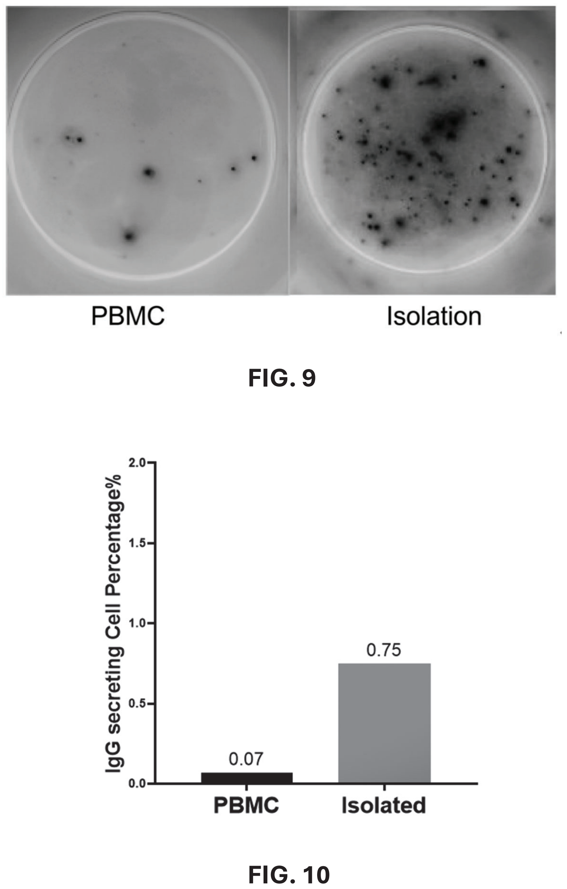

The specific antibodies and their concentrations in this embodiment were: CD3 monoclonal antibody 5 µg/mL, CD14 monoclonal antibody 10 µg/mL, CD11b monoclonal antibody 10 µg/mL, and CD16 monoclonal antibody 10 µg/mL. As shown in **FIGS. 9-10****,** relative to the cells before enrichment, the proportion of antibody-secreting B cells after enrichment increased by 10.7-fold.

### Example 10. High-Throughput Screening of Monoclonal Antibodies Secreted by Human Plasma B Cells

This example/embodiment details a high-throughput methodology for screening monoclonal antibodies secreted by human plasma B cells. The specific procedures are as follows:
(1) preparation of Antigen-Coated Magnetic Beads:
   Approximately 25 µL of uniformly resuspended streptavidin magnetic beads (10 mg/mL, 300 nm diameter) were washed three times with PBST buffer. The beads were resuspended in 200 µL PBST, and biotin-labeled antigen was added to a final concentration of 100 nM.
   Following a 30-minute incubation at room temperature, the supernatant was discarded. The beads were washed twice with PBST and resuspended in 200 µL DPBSB (DPBS + 0.5% w/v BSA) buffer for storage at 4°C.
(2) preparation of Human Plasma B Cell Suspension:
   Peripheral Blood Mononuclear Cells (PBMCs) were isolated from human peripheral blood using density gradient centrifugation with LYMPHOPREP^{™} reagent;
   CD38+ plasma cells were subsequently isolated from the PBMCs using a plasma cell enrichment kit and resuspended in 1 mL DPBSB on ice.
(3) pre-incubation of Plasma B Cells:
   Enriched plasma B cells were resuspended in DPBSB at 1 × 10⁷ cells/mL. Jackson Dylight-405 Goat Anti-Human IgG(H+L) and Dylight-405 F(ab')₂ Fragment Rabbit Anti-Human IgG-Fcy antibodies were added at a 1:100 volume ratio. After incubating for over 10 minutes at 4°C protected from light.

The cells were filtered through a 20 µm cell strainer. Cells were recovered via centrifugation at 500 × g for 5 minutes and resuspended in DPBSB on ice.
(4) preparation of droplet assay buffer:
   A mixture containing 816 µL Basal droplet assay buffer, 10 µL Jackson PE Goat Anti-Human IgG-Fcy antibody, and 4.1 µL NUCGREEN^{™} fluorescent dye was prepared. The droplet assay buffer was uniformly mixed and purified through a 0.22 µm filter.
(5) microdroplet Generation, Incubation, and Sorting:
   Taking 100 µL of the antigen-coated magnetic bead suspension from step (1), discarding the supernatant, uniformly resuspending the beads in 120 µL droplet assay buffer from step (4), then adding to injection port 1 of the DPBIO droplet generation chip;
   Taking 0.6 × 106 pre-blocked plasma B cells from step (3), uniformly resuspending in 120 µL droplet assay buffer from step (4), then adding to injection port 2;
   Taking 500 µL droplet generation oil, adding to injection port 3;

Using DPBIO droplet generator, air pressures were set to 2.7 psi (ports 1 and 2) and 5.4 psi (oil port) to co-encapsulate beads and cells.

The microdroplets were incubated at 37°C for 2 hours.

Following incubation, the microdroplets are introduced into a specialized droplet sorting chip for high-throughput evaluation. The fluidic dynamics within the chip are precisely regulated by a three-way air pressure system: 3.8 psi (sample flow)-3.5 psi (spacer flow)-10.0 psi (sheath flow). The droplets transit through the fluorescence detection window of the chip sequentially at a stabilized frequency of 200 Hz.

The 405 nm laser power is set to 10 mW and the 488 nm laser power is set to 10 mW. The gain parameters for the NUCGREEN^{™}, PE, and Dylight-405 fluorescence channels are each set to 620. Fluorescent signals are acquired as each droplet transits through the detection window. The system analyzes the maximum intensity value (peak signal) generated in each channel for every individual droplet.

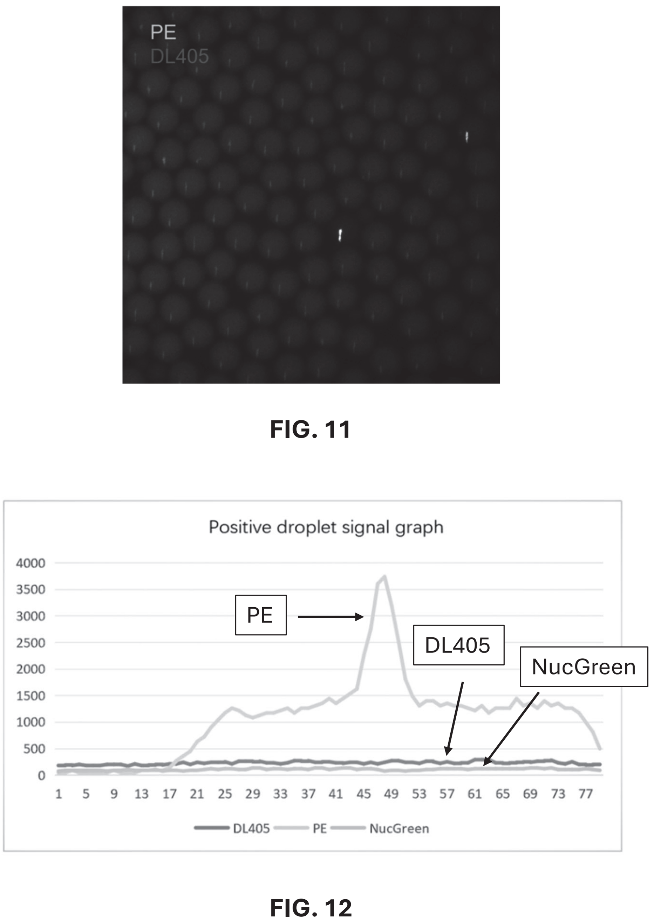

In this example, the fluorescence profiles of the generated microdroplets are illustrated in FIG. 11. Based on the established sorting criteria, target droplets are identified as those exhibiting a NUCGREEN^{™} signal < 400, a PE signal > 1900, and a Dylight-405 signal < 400. The results of this selection are shown in **FIG. 12**. As can be seen from the figure, there are positive droplets with PE fluorescence on the magnetic bead surface. Positive droplets are characterized by distinct PE fluorescence localized on the magnetic bead. Negative droplets exhibit Dylight-405 fluorescence on the cell surface but lack a PE signal. The imaging data is consistent with the signal curves, which display sharp peak signals in the PE channel and only baseline background signals in the Dylight-405 and NUCGREEN^{™} channels.

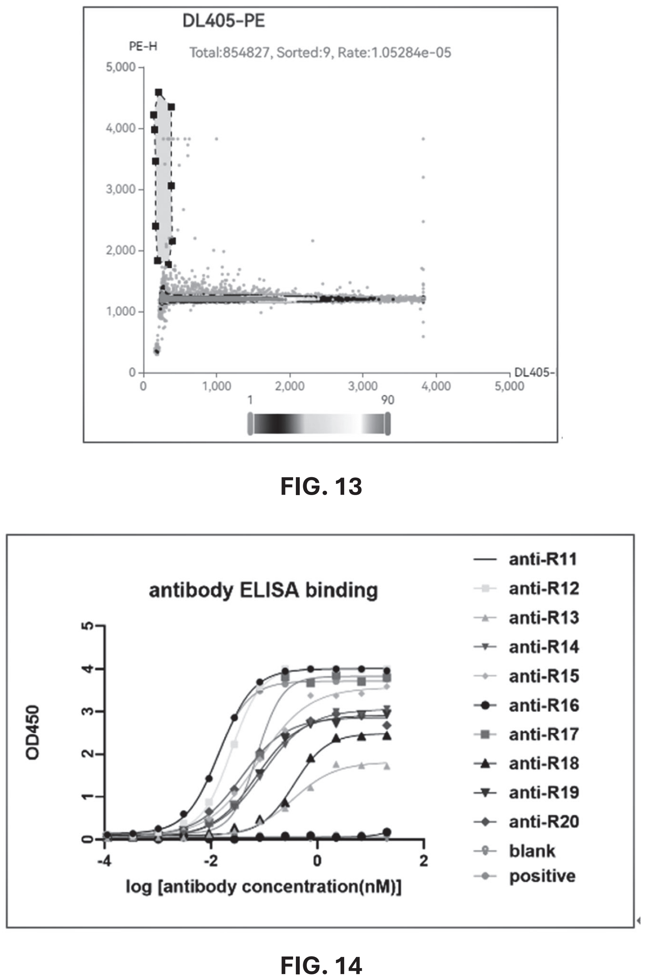

**FIG. 13** presents a scatter plot of the total droplet population and the identification results. While a substantial number of droplets show peak signals in the Dylight-405 channel-some of which coincide with PE signals-the system is designed to isolate specific targets. Specifically, positive droplets are selected only if their signal intensity shows a statistically significant difference in the PE channel but no statistically significant difference in the Dylight-405 channel, thereby significantly improving the sorting accuracy and true positive rate.
(6) enrichment and validation of monoclonal antibodies from positive cells:
The sorting voltage is configured to 1600 V with a duration of 1000 µs. A dielectrophoretic force is utilized to drive the identified positive droplets into a collection channel, where they are deposited into microwells containing cell lysis buffer and RNase inhibitors.

Following sorting, the DPBIO human single-cell BCR amplification and library preparation kit is used to amplify the B-cell receptor (BCR) genes from each positive cell. Paired light and heavy chain DNA sequences are successfully obtained through next-generation sequencing (NGS).

The functional binding ability of the antibodies produced from the screened droplets was verified via ELISA (targeting soluble antigens). The results, shown in **FIG. 14****,** confirm that 90% of the recovered antibodies possess high affinity for the target antigen.

### Example 11. Evaluation Effect of Non-Removal and Removal of Aggregation Particles in Droplet Assay Buffer on Droplet Sorting

816 µL of DPBIO droplet basal buffer was taken, and 10 µL of Jackson PE Goat Anti-Human IgG-Fcy antibody and 4.1 µL of NUCGREEN^{™} fluorescent dye were added. After mixing uniformly, the solution was filtered through a 0.22 µm filter membrane and placed on ice protected from light for later use. For the control group, the droplet detection buffer was used directly after preparation without centrifugation or filtration to remove aggregated particles.

120 µL of the droplet buffer was added to Inlet 1 and Inlet 2, respectively. 500 µL of droplet generation oil was taken and added to Inlet 3. The three-channel pressures on the DPBIO droplet generator were set to 2.7 psi, 2.7 psi, and 5.4 psi to form microdroplets. The microdroplets were incubated at 37°C for 2 hours. After incubation, aliquot of microdroplets were examined under fluorescence microscopy. The remaining microdroplets were introduced into a droplet sorting chip. The droplets sequentially passed through the fluorescence detection window of the chip at a frequency of 200 Hz under conditions where the sample flow, spacer flow, and carrier flow pressures were 3.8 psi, 3.5 psi, and 10.0 psi, respectively. The 405 nm laser power was set to 10 mW, and the 488 nm laser power was set to 10 mW. The gain parameters for the NUCGREEN^{™}, PE were set to 620. Signals in each fluorescence channel were collected as each droplet passed through the detection window, and the maximum intensity value of the signal for each droplet was analyzed.

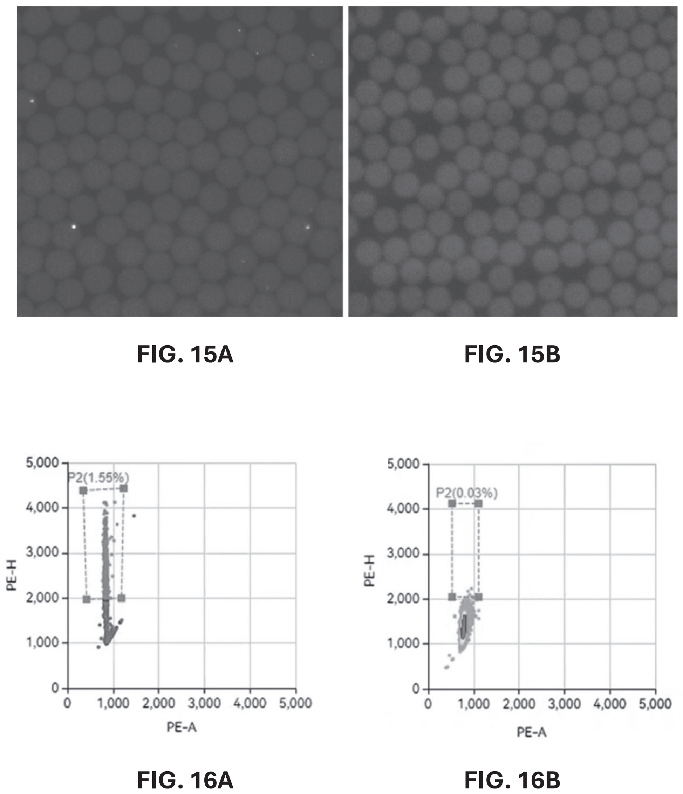

By observing the fluorescence images of the droplets and detecting the droplet fluorescence signals for both the filtered and unfiltered groups, it was demonstrated that filtration of the droplet buffer containing fluorescent antibodies is effective in reducing non-specific signals. **FIGS. 15A-15B** present the fluorescence images of the droplets in the PE channel obtained from the filtered group and the unfiltered group, showing that the droplets without filtration treatment contained fluorescent bright spots of varying particle sizes and uneven intensities, whereas the droplets subjected to filtration treatment were substantially free of fluorescent bright spots. The bright spots may come from the large aggregates of PE secondary detection antibodies or other artifacts. **FIGS. 16A-16B** present the fluorescence signals of the droplets in the PE channel obtained from the filtered group and the unfiltered group by droplet screening. It can be seen that the droplet samples treated with filtration exhibited substantially no non-target fluorescence signals, whereas the droplet samples without filtration treatment exhibited a large number of non-target fluorescence signals. The unwanted PE signals from detection secondary antibodies or other artifacts may be categorized as the false positive ones during droplet sorting.

### Example 12. Evaluation of Blocking/Non-Blocking of Plasma Cells or Reporter Cells Prior to Encapsulation

CHO reporter cells without the antigen expressed were prepared into a single-cell suspension and resuspended in DPBSB buffer at a concentration of 1×10⁷/mL. Jackson Goat Anti-Mouse IgG (H+L) antibody was added at a 1:200 volume ratio, and the mixture was incubated at 4°C protected from light for 10 minutes. Subsequently, Jackson Alexa-Fluor-647 F(ab)'2 Fragment Rabbit Anti-Mouse IgG-Fcy antibody was added at a 1:200 volume ratio, and the mixture was incubated at 4°C protected from light for >10 minutes. The pre-incubated reporter cells were filtered through a 20 µm filter, recovered by centrifugation at 500g for 5 minutes, resuspended in DPBSB, and placed on ice protected from light for later use. For control group, CHO reporter cells were directly filtered through a 20 µm filter without any secondary antibody incubation to block non-specific binding. The enriched mouse plasma B cells were blocked as CHO reporter cells and filtered through a 20 µm filter. For control group, plasma B cells were directly filtered through a 20 µm filter without blocking.

816 µL of DPBIO droplet basal buffer was taken, and 10 µL of Jackson PE Goat Anti-Mouse IgG-Fcy antibody and 4.1 µL of NUCGREEN^{™} fluorescent dye were added. After mixing uniformly, the solution was filtered through a 0.22 µm filter membrane and placed on ice protected from light for later use.

0.6×10⁶ of the plasma B cells pre-incubated with or without blocking secondary antibodies were taken, recovered by centrifugation, uniformly resuspended in 120 µL of the droplet buffer, and then added to Inlet 1 of a DPBIO droplet generation chip. 4.5×10⁶ of the reporter cells pre-incubated with or without blocking secondary antibodies were taken, recovered by centrifugation, uniformly resuspended in 120 µL of the droplet buffer, and then added to Inlet 2. 500 µL of droplet generation oil was taken and added to Inlet 3. The three-channel pressures on the DPBIO droplet generator were set to 2.7 psi, 2.7 psi, and 5.4 psi, respectively, causing the oil in Inlet 3 to co-encapsulate the plasma cells from Inlet 1 and the reporter cells from Inlet 2 to form microdroplets. The microdroplets were incubated at 37°C for 2 hours. After incubation was completed, aliquot of the microdroplets was examined under fluorescence microscopy.

The remaining microdroplets were introduced into a droplet sorting chip for droplet analysis. The droplets sequentially passed through the fluorescence detection window of the chip at a frequency of 200 Hz under conditions where the sample flow, spacer flow, and carrier flow pressures were 3.8 psi, 3.5 psi, and 10.0 psi, respectively. The 488 nm laser power was set to 10 mW, and the 635 nm laser power was set to 30 mW. The gain parameters for the NUCGREEN^{™}, PE, and AF647 channels were set to 620. The maximum signal intensity value in each fluorescence channel was collected for each droplet.

Since negative CHO reporter cells not expressing the target antigen were used, therefore, no true positive signals existed within the obtained droplets. By observing the fluorescence images of the droplets and detecting the droplet fluorescence signals for both the blocking and non-blocking groups, it was demonstrated that performing blocking treatment on the plasma cell samples is effective in reducing and marking non-specific cell signals.

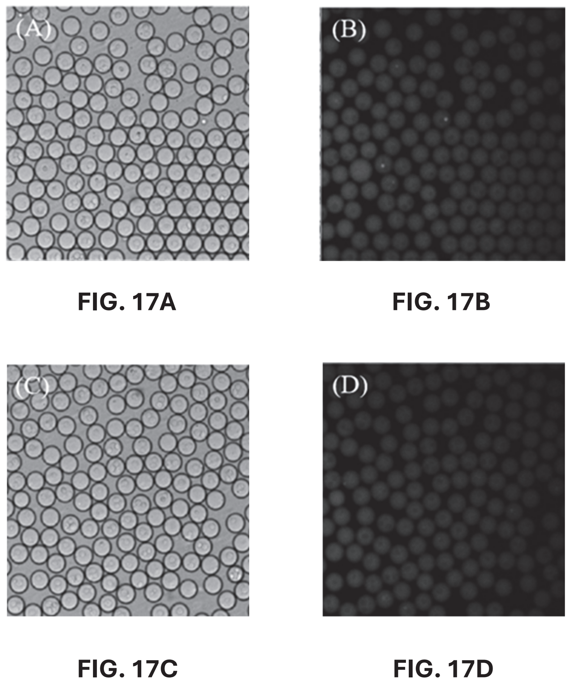

**FIGS. 17A-17D** present the images of the droplets in the bright field and the PE fluorescence channel for the blocking and non-blocking groups, showing that the droplets without blocking treatment contained PE fluorescence on the cell surface, whereas the PE fluorescence signal on the cell surface within the droplets subjected to blocking treatment was substantially reduced. **FIGS. 18A-18B** presents the fluorescence signals of the droplets analysis in the PE/AF647 channels for the blocking and non-blocking groups by the droplet sorter. It can be seen that the droplet samples treated with blocking exhibited substantially no strong PE signals but a large number of AF647 signals, whereas the droplet samples without blocking treatment exhibited a large number of non-target PE fluorescence signals. AF647 signals indicates a lot of non-specific binding occur during reporter cells or plasma cells blocking steps with AF647 secondary antibodies. The PE signals may come from non-specific binding of PE detection secondary antibodies to reporter cells or plasma B cells, which lead to false positive in droplet sorting.

From the results of Examples 11-12, it can be seen that the operations of subjecting cells to prior blocking treatment (pre-incubation) and filtering the droplet buffer containing fluorescent antibodies can reduce the interference of non-specific signals and non-target signals existing within the system, thereby improving the recognition rate of positive droplets and increasing the positive rate.

### Example 13. High-Throughput Single B-cell Antibody Screening from Rabbit Plasma B Cells with Bead Assay

This example/embodiment provides a method for high-throughput screening of monoclonal plasma cell-secreting antibodies from rabbit plasma B cells, specifically comprising:
(1) preparation of antigen-coated magnetic beads:
   25 µL of uniformly resuspended Beaver streptavidin magnetic beads (10 mg/mL, average diameter 300 nm) were taken, washed three times with PBST buffer, and resuspended in 200 µL of PBST. Biotin-labeled antigen was added to a final concentration of 100 nM, and the mixture was incubated at room temperature for 30 minutes. After incubation, the supernatant was discarded. The beads were washed twice with PBST, resuspended in 200 µL of DPBSB (DPBS + 0.5% BSA) buffer, and temporarily stored at 4°C for later use.
(2) enrichment and pre-incubation of plasma B cells from rabbit splenocytes:
   Rabbit plasma B cell enrichment was performed according to the method described in Example 7. The enriched plasma B cells were resuspended in DPBSB buffer at a concentration of 1×10⁷/mL. Jackson Goat Anti-Rabbit IgG (H+L) antibody and Jackson Goat Anti-Rabbit IgG-Fcy antibody were added respectively at a 1:100 volume ratio. The mixture was incubated at 4°C protected from light for >10 minutes. The pre-incubated plasma B cells were filtered through a 20 µm filter, recovered by centrifugation at 500g for 5 minutes, resuspended in DPBSB, and placed on ice protected from light for later use.
(3) preparation of droplet buffer:
   816 µL of DPBIO droplet basal buffer was taken, and 10 µL of Jackson PE Goat Anti-Rabbit IgG (H+L) antibody and 4.1 µL of NUCGREEN^{™} fluorescent dye were added. After mixing uniformly, the solution was filtered through a 0.22 µm filter membrane and placed on ice protected from light for later use.
(4) preparation, incubation, and sorting of microdroplets:
   **FIG. 19** is a schematic diagram illustrating the structural components of a picoliter droplet during microfluidic screening.

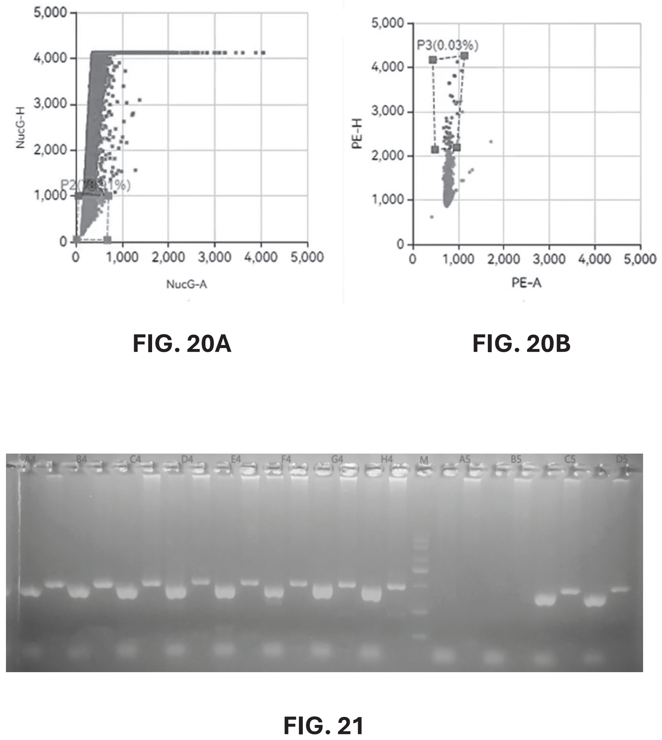

100 µL of the antigen-coated magnetic bead suspension from step (1) was taken, the supernatant was discarded, and the beads were uniformly resuspended in 120 µL of the droplet buffer from step (3), then added to Inlet 1 of a DPBIO droplet generation chip. 0.6×10⁶ of the plasma B cells pre-incubated in step (2) were taken, uniformly resuspended in 120 µL of the droplet buffer from step (3), then added to Inlet 2. 500 µL of droplet generation oil was taken and added to Inlet 3. The three-channel pressures on the DPBIO droplet generator were set to 2.7 psi, 2.7 psi, and 5.4 psi, respectively, causing the oil in Inlet 3 to co-encapsulate the magnetic beads from Inlet 1 and the plasma cells from Inlet 2 to form microdroplets. The microdroplets were incubated at 37°C for 2 hours. After incubation was completed, the microdroplets were introduced into a droplet sorting chip. The droplets sequentially passed through the fluorescence detection window of the chip at a frequency of 200 Hz under conditions where the sample flow, spacer flow, and carrier flow pressures were 3.8 psi, 3.5 psi, and 10.0 psi, respectively. The 488 nm laser power was set to 10 mW. The gain parameters for the NUCGREEN^{™}, and PE were set to 620. Signals generated in each fluorescence channel were collected as each droplet passed through the detection window, and the maximum intensity value of the signal for each droplet was analyzed. In this example/embodiment, according to the droplet sorting criteria, droplets with a NUCGREEN^{™} signal value < 400, and a PE signal value > 1900 were selected as positive droplets. **FIGS. 20A-20B** show the droplet signal scatter plot and positive droplet identification results of this example/embodiment. Through the above-mentioned sorting criteria, this example/embodiment was able to select droplets that had a significant difference in signal intensity only in the PE channel, but no significant difference in the NUCGREEN^{™} channel to remove dead cells, as the positive droplets of this embodiment, thereby increasing the positive rate of sorting.

### (5) enrichment and validation of positive droplets:

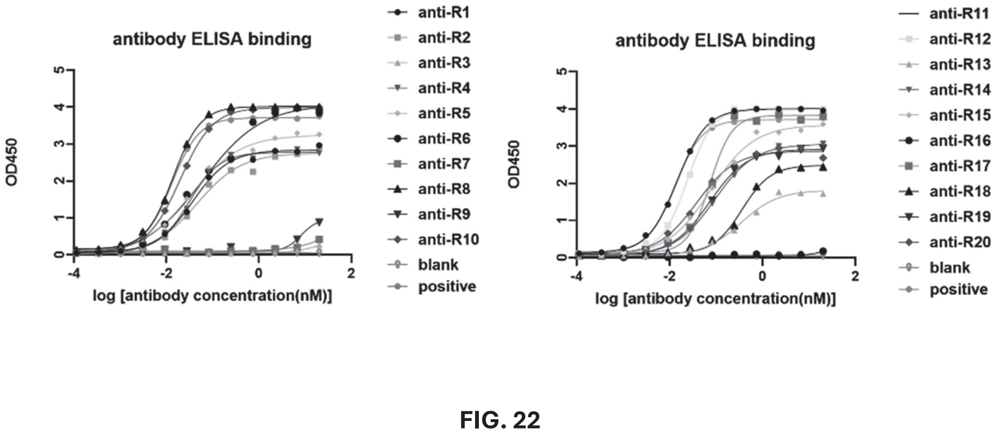

The sorting voltage was set to 1600 V with a duration of 1000 µs, utilizing dielectrophoretic force to drive the positive droplets into the positive collection channel, where they fell into microwells containing cell lysis buffer and RNase inhibitor. After sorting was completed, a DPBIO rabbit single-cell BCR amplification and library construction kit was used to obtain BCR amplicons of each positive cell, as shown in **FIG. 21****,** and paired light and heavy chain DNA sequences were obtained through Sanger sequencing. **FIG. 22** shows the ELISA verification results of the antibodies obtained from the positive droplets screened in this embodiment (against soluble antigen targets), showing that 90% of the antibodies possessed affinity for the target antigen.

### Example 14. High-Throughput Single B-cell Antibody Screening from Mouse Plasma B cells with Reporter Cell Assay

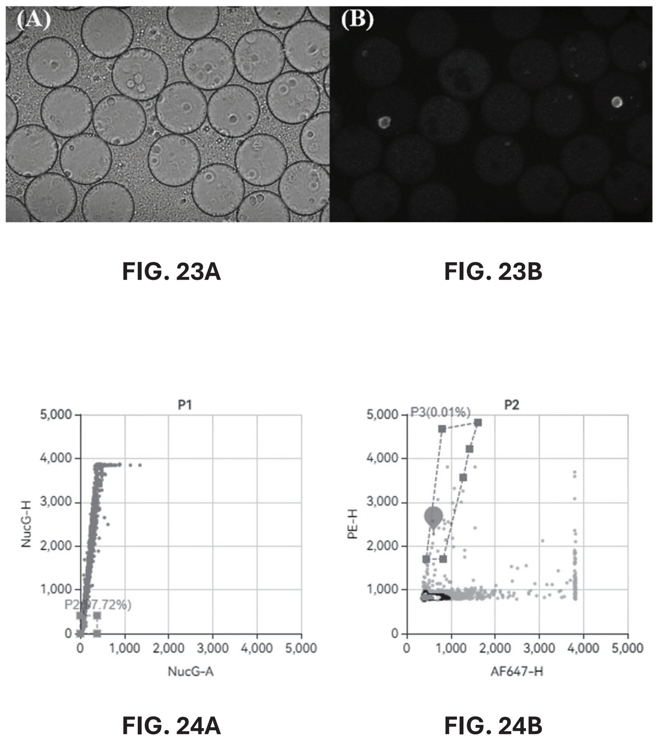

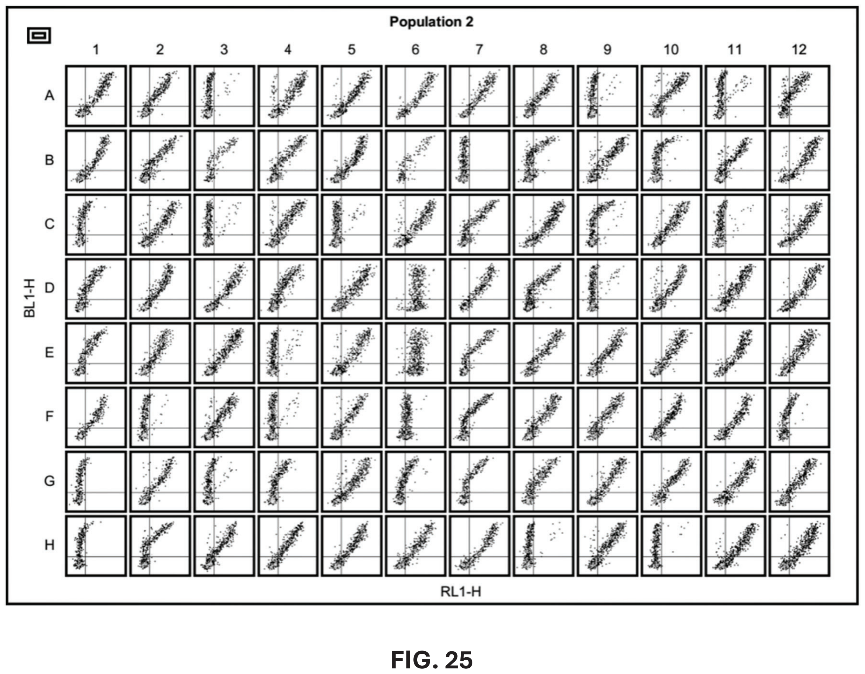

This example/embodiment provides a method for high-throughput screening of monoclonal plasma cell-secreting antibodies from mouse plasma B cells, specifically comprising:
(1) preparation of mouse spleen plasma B cell suspension:
   Fresh mouse spleens were harvested, placed in a 40 µm cell strainer and 1640 medium, and a spleen cell suspension was obtained via grinding. CD138+ cells and plasma B cells were isolated from said mouse spleen cells using a Mouse Plasma Cell Enrichment/Release Kit (STEMCELL^{™}), resuspended in 1 mL of DPBSB, and placed on ice for later use.
(2) pre-incubation of reporter cells
   CHO reporter cells were prepared into a single-cell suspension and resuspended in DPBSB buffer at a concentration of 1×10⁷/mL. Jackson Goat Anti-Mouse IgG (H+L) antibody was added at a 1:200 volume ratio, and the mixture was incubated at 4°C protected from light for 10 minutes. Subsequently, Jackson Alexa-Fluor-647 F(ab')2 Fragment Rabbit Anti-Mouse IgG-Fcy antibody was added at a 1:200 volume ratio, and the mixture was incubated at 4°C protected from light for >10 minutes. The pre-incubated reporter cells were filtered through a 20 µm filter, recovered by centrifugation at 500g for 5 minutes, resuspended in DPBSB, and placed on ice protected from light for later use.
(3) pre-incubation of plasma B cells:
   The enriched plasma B cells were resuspended in DPBSB buffer at a concentration of 1×10⁷/mL. Jackson Goat Anti-Mouse IgG (H+L) antibody and Jackson Alexa-Fluor-647 F(ab')2 Fragment Rabbit Anti-Mouse IgG-Fcy antibody were added respectively at a 1:100 volume ratio. The mixture was incubated at 4°C protected from light for >10 minutes. The pre-incubated plasma B cells were filtered through a 20 µm filter, recovered by centrifugation at 500g for 5 minutes, resuspended in DPBSB, and placed on ice protected from light for later use.
(4) preparation of droplet buffer:
   816 µL of DPBIO droplet basal buffer was taken, and 10 µL of Jackson PE Goat Anti-Mouse IgG-Fcy antibody and 4.1 µL of NUCGREEN^{™} fluorescent dye were added. After mixing uniformly, the solution was filtered through a 0.22 µm filter membrane and placed on ice protected from light for later use.
(5) preparation, incubation, and sorting of microdroplets:
   0.6×10⁶ of the plasma B cells pre-incubated in step (3) were taken, recovered by centrifugation, uniformly resuspended in 120 µL of the droplet buffer from step (4), and then added to Inlet 1 of a DPBIO droplet generation chip. 4.5×10⁶ of the reporter cells pre-incubated in step (2) were taken, recovered by centrifugation, uniformly resuspended in 120 µL of the droplet buffer from step (4), and then added to Inlet 2. 500 µL of droplet generation oil was taken and added to Inlet 3. The three-channel pressures on the DPBIO droplet generator were set to 2.7 psi, 2.7 psi, and 5.4 psi, respectively, causing the oil in Inlet 3 to co-encapsulate the plasma cells from Inlet 1 and the reporter cells from Inlet 2 to form microdroplets. The microdroplets were incubated at 37°C for 2 hours . After incubation was completed, the microdroplets were introduced into a droplet sorting chip. The droplets sequentially passed through the fluorescence detection window of the chip at a frequency of 200 Hz under conditions where the sample flow, spacer flow, and carrier flow pressures were 3.8 psi, 3.5 psi, and 10.0 psi, respectively. The 488 nm laser power was set to 10 mW, and the 635 nm laser power was set to 30 mW. The gain parameters for the NUCGREEN^{™}, PE, and AF647 channels were set to 620. The maximum signal intensity value in each fluorescence channel was collected for each droplet. According to the droplet sorting criteria, droplets with a NUCGREEN^{™} signal value < 400, a PE signal value > 1800, and an AF647 signal value showing no significant difference from the background signal were selected as positive droplets. In this embodiment, the bright-field images and PE/AF647 fluorescence images of the droplets are shown in **FIGS. 23A-23B****.** It shows that some droplets co-encapsulated a single plasma cell and several reporter cells. On the surface of some co-encapsulated reporter cells, distinct fluorescence signals in the PE channel were presented, representing the binding signal of positive secretory antibodies; simultaneously, AF647 signals from the blocking antibody were also present on the cell surface within some droplets. Further, **FIGS. 24A-****24B** show the signal scatter plots and positive droplet identification results for the droplets of this embodiment in the NUCGREEN^{™} channel and the PE/AF647 channel. **FIG. 24A** shows the presence of a group of droplets exhibiting strong NUCGREEN^{™} fluorescence due to containing dead cells, while the proportion of droplets containing no dead cells was >97%. **FIG. 24B** shows the presence of a large number of AF647 positive droplets, some of which were simultaneously PE positive, which may originate from droplets multi-encapsulating negative and positive plasma cells, cells where the blocking effect was not fully realized, or false positive signals gradually accumulated during incubation. According to the screening criteria for positive droplets in this embodiment-specifically selecting droplets that have a significant difference in the PE channel compared to the droplet background signal, but no significant difference in the AF647 channel-non-target droplets such as those containing cell surface false positive signals or droplets multi-encapsulating negative and positive plasma cells can be excluded, possessing the advantage of high accuracy. In this embodiment, due to the existence of fluorescence crosstalk, PE positive droplets exhibited a slight signal drift in the AF647 channel, which did not affect positive droplet identification.
(6) enrichment and validation of positive droplets:
   The sorting voltage was set to 1600 V with a duration of 1000 µs, utilizing dielectrophoretic force to sort single positive droplets into the positive collection channel, where they fell into microwells containing cell lysis buffer and RNase inhibitor. After sorting was completed, a DPBIO mouse single-cell BCR amplification and library construction kit was used to perform BCR amplification on each positive cell, and paired light and heavy chain DNA sequences were obtained through Sanger sequencing. **FIG. 25** shows the FACS verification results of the antibodies obtained from the positive droplets screened in this embodiment (against membrane protein antigen targets), showing that over 80% of the antibodies possessed affinity for the membrane antigen on the surface of the reporter cells (RL1 and BL1 double-positive samples).

### ENUMERATED EMBODIMENTS

1. A method for enriching and identifying antigen-specific antibody-secreting cells, comprising:
   (a) contacting a heterogeneous immune cell population with at least one depletion antibody that specifically binds non-B-lineage cells of the same species, wherein the at least one depletion antibodies is conjugated to a tag, optionally wherein the tag is selected from biotin, fluorophore, peptide tag, affinity tag, chemical label, and any combination thereof, thereby producing depletion-antibody-labeled cells;
   (b) removing the depletion-antibody-labeled cells from the heterogeneous immune cell population by adding affinity beads capable of specifically binding the tag to the heterogenous immune cell population, optionally wherein the beads comprise anti-biotin beads, anti-fluorophore beads, anti-tag beads, streptavidin beads, avidin beads, Ni-NTA beads, glutathione beads, or any combination thereof, thereby producing an enriched population of B cells or plasma B cells, optionally wherein at least 50% (e.g., at least 60%, 70%, 75%, 80%, 85%, 90%, or 95%) of the cells in the population are B cells or plasma B cells;
   (c) co-encapsulating the enriched population of B cells or plasma B cells into microfluidic droplets together with (A):
      (i) antigen-coated beads; and/or
      (ii) antigen-expressing reporter cells; and

      with at least one detection antibody capable of binding immunoglobulin secreted by the enriched population of B cells or plasma B cells, optionally wherein the at least one detection antibody is labeled (*e.g*., fluorescently labeled); or
      together with (B):
         (iii) an antigen (*e.g.,* a labeled (*e.g.,* fluorescently labeled) antigen) and immunoglobulin-capture (*e.g.,* IgG-capture) antibody-coated beads; and
      optionally with at least one detection antibody capable of binding immunoglobulin secreted by the enriched population of B cells or plasma B cells, *e.g.,* to differentiate IgG and IgM, optionally wherein the at least one detection antibody is labeled (*e.g.,* fluorescently labeled),optionally wherein 15%-25% (*e.g.,* about 20%) of the encapsulated microfluidic droplets comprise one or two B cells or plasma B cells (*e.g.,* one B cell or plasma B cell); 85%-95% (*e.g.,* about 90%) of the encapsulated microfluidic droplets comprise one or two antigen-expressing reporter cells (*e.g.,* one antigen-expressing reporter cell); and/or 10%-20% of the encapsulated microfluidic droplets comprise (a) one or two B cells or plasma B cells (*e.g.,* one B cell or plasma B cell); and (b) one or two antigen-expressing reporter cells (*e.g.,* one antigen-expressing reporter cell);
   (d) incubating the droplets to allow antibody secretion;
   (e) detecting droplets exhibiting a localized fluorescence signal indicative of antigen-specific antibody binding;
   (f) sorting positive droplets;
   (g) recovering antibody-secreting cells; and
   (h) optionally, isolating and/or amplifying nucleic acids encoding immunoglobulin heavy and/or light chains, or a fragment thereof, from the antibody-secreting cells.
2. The method of embodiment 1, wherein the tag is selected from biotin, FITC, PE, APC, Cy5, an HA-tag, a FLAG-tag, a His-tag, a GST-tag, a SNAP-tag, a CLIP-tag, DIG, DNP, or a derivative thereof.
3. The method of embodiment 1 or 2, wherein the affinity beads are 0.1-10 µm magnetic particles coated with an anti-tag antibody, an affinity ligand, a peptide binder, a metal, a polymer, or any combination thereof.
4. The method of any one of embodiments 1-3, wherein the at least one depletion antibody is chosen according to the species of origin and comprises an antibody recognizing one or more of: a T-cell marker, an NK-cell marker, a monocyte marker, a macrophage marker, a dendritic-cell marker, a granulocyte marker, or an IgM-positive naïve B-cell marker, optionally wherein the at least one depletion antibody comprises a plurality of depletion antibodies.
5. The method of embodiment 4, wherein the species is selected from rabbit, rat, hamster, bovine, ovine, canine, feline, camelid, llama, alpaca, non-human primate, or any combination thereof.
6. The method of any one of embodiments 1-5, further comprising treating the enriched population of B cells or plasma B cells, the antigen-coated beads, the antigen-expressing reporter cells, and/or the antigen with an immunoglobulin-capture antibody-coated beads, with a blocking antibody or an FcR-blocking reagent to reduce non-specific interactions, optionally wherein the blocking antibody or reagent is labeled (*e.g.,* fluorescently labeled).
7. The method of any one of embodiments 1-6, wherein the detection further comprises using a viability dye, optionally wherein the viability dye is selected from NUCGREEN^{™}, NUCRED^{™}, EVAGREEN^{™}, a SYTOX^{™} dye, or an equivalent thereof, and wherein droplets containing dead cells are excluded.
8. The method of any one of embodiments 1-7, wherein the antigen-coated beads comprise polystyrene, silica, agarose, dextran, polymer, hydrogel, or magnetic beads, optionally wherein the antigen-coated beads have diameters of 20 nm to 50 µm.
9. The method of any one of embodiments 1-8, wherein the antigen-expressing reporter cells express a membrane protein, a multi-pass receptor, a GPCR, an ion channel, a viral glycoprotein, or another conformational antigen.
10. The method of any one of embodiments 1-9, wherein a droplet is classified as positive when:
   (a) the detection antibody generates a signal (*e.g.,* fluorescence signal) that exceeds a predetermined threshold; and
   (b) the signal (*e.g.,* fluorescence signal) generated by dead cells is at background level; and
   (c) optionally, the signal (*e.g.,* fluorescence signal) generated by the blocking antibody is at background level.
11. The method of any one of embodiments 1-10, wherein the sorting is performed by dielectrophoresis, acoustics, pneumatic valves, mechanical deflection, optical sorting, or surface-energy-based sorting.
12. The method of any one of embodiments 1-11, further comprising RT-PCR amplification (*e.g.,* single B cell BCR amplification) of variable heavy- and light-chain sequences, and optionally sequencing (*e.g.,* bulk BCR sequencing) the sequences, *e.g.,* using Sanger sequencing, Illumina sequencing, PacBio sequencing, Oxford Nanopore sequencing, or a combination thereof.
13. A method for enriching single B cells (*e.g.,* rabbit or camelid single B cells), comprising the following steps:
   S1. incubating a single-cell suspension (*e.g.,* a rabbit or camelid single-cell suspension) with a biotinylated specific antibody composition, such that antibodies in the composition specifically bind to non-target cells to be removed; and
   S2. adding streptavidin-coated magnetic beads to the cell suspension from S1, and subjecting the mixture to magnetic separation to remove the non-target cells bound to the antibodies, thereby obtaining an enriched population of single B cells (e.g., rabbit or camelid single B cells);
   wherein said antibody composition comprises one or more antibodies selected from the group consisting of an anti-T lymphocyte antibody, an anti-CD4 antibody, an anti-CD8 antibody, an anti-CD11b antibody, an anti-IgM antibody, an anti-CD11c antibody, and an anti-CD123 antibody.
14. The method for enriching single B cells (e.g., rabbit or camelid single B cells) according to embodiment 13, wherein, in said cell suspension, the concentrations of the specific antibodies are: T lymphocyte antibody 1 µg/mL-30 µg/mL, CD4 monoclonal antibody 1 µg/mL-30 µg/mL, CD8 monoclonal antibody 1 µg/mL-30 µg/mL, CD11b monoclonal antibody 1 µg/mL-50 µg/mL, IgM monoclonal antibody 1 µg/mL-40 µg/mL, CD11c monoclonal antibody 1 µg/mL-30 µg/mL, and CD123 monoclonal antibody 1 µg/mL-30 µg/mL.
15. The method for enriching single B cells (e.g., rabbit or camelid single B cells) according to embodiment 12, characterized in that, in said cell suspension, the concentrations of the specific antibodies are: T lymphocyte antibody 5 µg/mL-30 µg/mL, CD4 monoclonal antibody 5 µg/mL-30 µg/mL, CD8 monoclonal antibody 5 µg/mL-30 µg/mL, CD11b monoclonal antibody 10 µg/mL-50 µg/mL, IgM monoclonal antibody 10 µg/mL-40 µg/mL, CD11c monoclonal antibody 5 µg/mL-30 µg/mL, and CD123 monoclonal antibody 5 µg/mL-30 µg/mL.
16. The method for enriching single B cells (e.g., rabbit or camelid single B cells) according to embodiment 13, characterized in that the antibodies are specific for rabbit or camelid antigens, wherein the CD4 monoclonal antibody is an anti-rabbit or camelid CD4 monoclonal antibody, the CD8 monoclonal antibody is an anti-rabbit or camelid CD8 monoclonal antibody, the CD11b monoclonal antibody is an anti-human, rabbit, or camelid CD11b monoclonal antibody, the IgM monoclonal antibody is an anti-rabbit or camelid IgM monoclonal antibody, the CD11c monoclonal antibody is a mouse anti-human, rabbit, or camelid CD11c monoclonal antibody, and the CD123 monoclonal antibody is a mouse anti-human, rabbit, or camelid CD123 monoclonal antibody.
17. The method for enriching single B cells (*e.g.,* rabbit or camelid single B cells) according to any one of embodiments 13-16, characterized in that said specific antibodies are a combination of at least 4 antibodies selected from an T lymphocyte antibody, CD4 monoclonal antibody, CD8 monoclonal antibody, CD11b monoclonal antibody, IgM monoclonal antibody, CD11c monoclonal antibody, and CD123 monoclonal antibody.
18. The method for enriching single B cells (*e.g.,* rabbit or camelid single B cells) according to embodiment 13, characterized in that the tissue source of rabbit or camelid single cells comprises rabbit or camelid spleen, and rabbit or camelid peripheral blood.
19. A microfluidic screening method for single B cells (*e.g*., rabbit or camelid single B cells), comprising:
   (a) obtaining an enriched single B cell suspension (*e.g.*, rabbit or camelid single B cell suspension) according to any one of embodiments 13-18;
   (b) providing antigen-coated microbeads by coating the antigen protein onto the surface of microbeads;
   (c) encapsulating the single B cell suspension (e.g., rabbit or camelid single B cell suspension) and the antigen-coated microbeads into a plurality of nanoliter or picoliter droplets using a microfluidic device, wherein the cells and microbeads are suspended in a buffer containing a detection antibody carrying a first fluorescent label;
   (d) incubating the droplets to allow the single B cells (e.g., rabbit or camelid single B cells) to secrete target specific antibodies, which form a complex with the antigen-coated microbeads and the detection antibody within the droplets;
   (e) detecting multi-channel fluorescent signals from the incubated droplets and sorting target droplets based on a fluorescent signal value; and
   (f) recovering target rabbit single B cells (e.g., rabbit or camelid single B cells) from the sorted droplets.
20. The microfluidic screening method for single B cells (e.g., rabbit or camelid single B cells) according to embodiment 19, characterized in that said buffer further comprises a dead cell fluorescent dye, and the fluorescent signal generated by said first fluorescent label is different from the fluorescent signal generated by the dead cell fluorescent dye.
21. The microfluidic screening method for single B cells (e.g., rabbit or camelid single B cells) according to embodiment 19 or 20, characterized by further comprising performing reverse transcription and PCR amplification on the screened target single B cells (e.g., rabbit or camelid single B cells), and obtaining the corresponding antibodies expressed by the B cells (e.g., rabbit or camelid B cells) through sequencing and expression verification.
22. A method for high-throughput screening monoclonal antibodies secreted by single plasma cells, characterized in that the method comprises:
   (1) providing a plurality of nanoliter or picoliter droplets, each droplet comprising:
      a. no more than one antibody-secreting cell;
      b. microbeads coated with a target antigen, or at least one reporter cell expressing the target antigen on cell surface;
      c. a detection antibody and/or a dead cell fluorescent dye;
   (2) incubating the plurality of droplets under suitable conditions to allow the antibody-secreting cell to secrete the target antibody, thereby obtaining incubated droplets;
   (3) passing the incubated droplets individually through a droplet sorting chip to perform multi-fluorescent signal detection, and sorting and enriching target droplets based on the fluorescent signal values of the droplets;

   wherein in step (1), the antibody-secreting cells are pre-incubated with a blocking antibody; and the reporter cell expressing the target antigen is pre-incubated with a blocking antibody;
   wherein said blocking antibody specifically binds to the target antibody; said detection antibody specifically binds to the target antibody; and said blocking antibody cannot be recognized or bound by said detection antibody; wherein the detection antibody carries a first fluorescent label, and the fluorescent signal produced by the first fluorescent label is distinct from the fluorescent signal produced by the dead cell fluorescent dye.
23. The method for high-throughput screening monoclonal antibodies secreted by single plasma cells according to embodiment 22, characterized in that the target droplets comprise:
   (i) droplets whose fluorescent signal value in the detection antibody fluorescent channel shows a statistically significant difference from the droplet background fluorescent value; and (ii) droplets whose fluorescent signal value in the dead cell dye fluorescent channel shows no statistically significant difference from the droplet background fluorescent value; or
   the target droplets comprise:
   1) droplets whose fluorescent signal value in the detection antibody fluorescent channel is at least 1.2 times the droplet background fluorescent value; and 2) droplets whose fluorescent signal value in the dead cell dye fluorescent channel is less than or equal to 1.1 times the droplet background fluorescent value.
24. The method for high-throughput screening monoclonal antibodies secreted by single plasma cells according to embodiment 22, characterized in that the blocking antibody carries a second fluorescent label, and the fluorescent signal produced by the second fluorescent label is distinct from the fluorescent signal produced by the first fluorescent label.
25. The method for high-throughput screening monoclonal antibodies secreted by single plasma cells according to embodiment 24, characterized in that the target droplets further comprise: (iii) droplets whose fluorescent signal value in the blocking antibody fluorescent channel shows no statistically significant difference from the droplet background fluorescent value; or the target droplets further comprise: 3) droplets whose fluorescent signal value in the blocking antibody fluorescent channel shows no statistically significant difference from the droplet background fluorescent value.
26. The method for high-throughput screening monoclonal antibodies secreted by single plasma cells according to embodiment 23 or 25, characterized in that the statistically significant difference refers to a p-value < 0.05; and the no statistically significant difference refers to a p-value > 0.1.
27. The method for high-throughput screening monoclonal antibodies secreted by single plasma cells according to embodiment 22, characterized in that the preparation of the droplets comprises the following steps:
   S1. preparing the antibody-secreting cells into a single-cell suspension, adding a blocking antibody for a blocking treatment, and washing the cells after the blocking treatment to remove excess blocking antibody, thereby obtaining a single-cell suspension of blocked antibody-secreting cells;
   S2. preparing the reporter cells expressing the target antigen into a single-cell suspension, adding a blocking antibody for a blocking treatment, and washing the cells after the blocking treatment to remove excess blocking antibody, thereby obtaining a single-cell suspension of blocked reporter cells; or coating the target antigen on the microbeads to obtain antigen-coated microbeads;
   S3. preparing a buffer solution containing the detection antibody and/or the dead cell fluorescent dye, and removing large particulate fluorescent substances from the buffer solution via filtration or high-speed centrifugation to obtain a filtered droplet assay buffer;
   S4. resuspending the blocked antibody-secreting cells and the blocked reporter cells respectively in the filtered droplet assay buffer, and mixing the resuspended suspensions to generate a plurality of nanoliter or picoliter droplets; or, resuspending the blocked antibody-secreting cells and the antigen-coated microbeads respectively in the filtered droplet assay buffer, and mixing the resulting suspensions to generate a plurality of nanoliter or picoliter droplets.
28. The method for high-throughput screening monoclonal antibodies secreted by single plasma cells according to embodiment 27, characterized in that in step S3, the filter membrane used for filtration is a bacterial filter membrane of 0.45 µm or below; and the centrifugal force for high-speed centrifugation is at least 8000 × g.
29. The method for high-throughput screening monoclonal antibodies secreted by single plasma cells according to embodiment 22, characterized in that the blocking antibody and the detection antibody are each independently selected from at least one of a monoclonal antibody and a polyclonal antibody.
30. The method for high-throughput screening monoclonal antibodies secreted by single plasma cells according to embodiment 24, wherein:
   (1) the first fluorescent label comprises any one fluorescent group selected from the group consisting of Alexa Fluor 488, Alexa Fluor 647, PE, and BV421;
   (2) the second fluorescent label comprises any one fluorescent group selected from the group consisting of Alexa Fluor 488, Alexa Fluor 647, PE, and BV421; and
   (3) the fluorescent signal produced by the second fluorescent label is distinct from the fluorescent signal produced by the first fluorescent label.
31. A method for enriching a population of B cells, the method comprising:
   (a) contacting a heterogeneous immune cell population with a plurality of depletion antibodies that specifically bind non-B-lineage cells of the same species, thereby producing depletion-antibody-labeled cells, wherein the plurality of depletion of antibodies are coupled (e.g., conjugated) to a tag, and wherein the tag is biotin, fluorophore, a peptide tag, an affinity tag, a chemical label, or a combination thereof; and
   (b) removing the depletion-antibody-labeled cells from the heterogeneous immune cell population by adding affinity beads capable of specifically binding the tag to the heterogeneous immune cell population, thereby producing an enriched population of B cells, optionally wherein at least 50% (e.g., at least 60%, 70%, 75%, 80%, 85%, 90%, or 95%) of the cells in the population are B cells,
   wherein the plurality of antibodies targeting T cells, monocytes, DCs, macrophage, IgM⁺ naïve B cells, and IgD⁺ B cells.
32. The method of embodiment 31, wherein the plurality of antibodies comprises an anti-T lymphocyte antibody, an anti-CD4 antibody, an anti-CD8 antibody, an anti-CD11b antibody, an anti-IgM antibody, an anti-CD11c antibody, an anti-IgD antibody, and an anti-CD123 antibody.
33. The method of embodiment 31, wherein the tag is selected from biotin, FITC, PE, APC, Cy5, an HA-tag, a FLAG-tag, a His-tag, a GST-tag, a SNAP-tag, a CLIP-tag, DIG, DNP, or a derivative thereof.
34. The method of any one of embodiments 31-33, wherein the affinity beads are 0.1-10 µm magnetic particles coated with an anti-tag antibody, an affinity ligand, a peptide binder, a metal, a polymer, or any combination thereof.
35. The method of any one of embodiments 31-34, wherein the species is selected from rabbit, rat, hamster, bovine, ovine, canine, feline, camelid, llama, alpaca, non-human primate, or any combination thereof.
36. A method for identifying an antigen-specific antibody-secreting cell, the method comprising:
   (a) providing microfluidic droplets co-encapsulated with a population of B cells (e.g., an enriched population of B cells, *e.g.*, as described herein) together with (A):
      (i) antigen-coated beads; and/or
      (ii) antigen-expressing reporter cells; and

      with at least one detection antibody capable of binding immunoglobulin secreted by the population of B cells; or
      together with (B):
         (iii) a labeled antigen (e.g., fluorescently labeled) and immunoglobulin-capture antibody-coated beads; and
      optionally, with at least one detection antibody capable of binding immunoglobulin secreted by the population of B cells, *e.g.,* to differentiate IgG and IgM,
      wherein the B cells, the antigen-coated beads, the antigen-expressing reporter cells, and/or the immunoglobulin-capture antibody-coated beads, have been treated with a blocking antibody or an FcR-blocking reagent to reduce non-specific interactions;
   (b) incubating the droplets to allow antibody secretion;
   (c) detecting droplets exhibiting a localized fluorescence signal indicative of antigen-specific antibody binding;
   (d) sorting positive droplets; and
   (e) recovering antibody-secreting cells from the positive droplets.
37. The method of embodiment 36, wherein the detection further comprises using a viability dye, optionally wherein the viability dye is selected from NUCGREEN^{™}, NUCRED^{™}, EVAGREEN^{™}, a SYTOX^{™} dye, or an equivalent thereof, and wherein droplets containing dead cells are excluded.
38. The method of embodiment 36 or 37, wherein the antigen-coated beads comprise polystyrene, silica, agarose, dextran, polymer, hydrogel, or magnetic beads, optionally wherein the antigen-coated beads have diameters of 20 nm to 50 µm.
39. The method of any one of embodiments 36-38, wherein the antigen-expressing reporter cells express a membrane protein, a multi-pass receptor, a GPCR, an ion channel, a viral glycoprotein, or another conformational antigen.
40. The method of any one of embodiments 36-39, wherein a droplet is classified as positive when:
   (a) the detection antibody generates a signal (e.g., fluorescence signal) that exceeds a predetermined threshold; and
   (b) the signal (e.g., fluorescence signal) generated by dead cells is at background level; and
   (c) optionally, the signal (e.g., fluorescence signal) generated by the blocking antibody is at background level.
41. The method of any one of embodiments 36-40, wherein the sorting is performed by dielectrophoresis, acoustics, pneumatic valves, mechanical deflection, optical sorting, or surface-energy-based sorting.
42. The method of any one of embodiments 36-41, further comprising amplifying nucleic acids encoding immunoglobulin heavy and/or light chains, or a fragment thereof, from the antibody-secreting cells.
42. The method of embodiment 41, further comprising sequencing the amplified nucleic acid, *e.g.,* using Sanger sequencing, Illumina sequencing, PacBio sequencing, Oxford Nanopore sequencing, or a combination thereof.

### INCORPORATION BY REFERENCE

All publications, patents, and Accession numbers mentioned herein are hereby incorporated by reference in their entirety as if each individual publication or patent was specifically and individually indicated to be incorporated by reference.

### EQUIVALENTS

While specific embodiments of the subject disclosure have been discussed, the above specification is illustrative and not restrictive. Many variations of the disclosure will become apparent to those skilled in the art upon review of this specification and the claims below. The full scope of the disclosure should be determined by reference to the claims, along with their full scope of equivalents, and the specification, along with such variations.

## Claims

1. A method for enriching and identifying an antigen-specific antibody-secreting cell, the method comprising:
(a) contacting a heterogeneous immune cell population with at least one depletion antibody that specifically binds non-B-lineage cells of the same species, thereby producing depletion-antibody-labeled cells, wherein the at least one depletion antibody is conjugated to a tag, and wherein the tag is biotin, fluorophore, a peptide tag, an affinity tag, a chemical label, or a combination thereof;
(b) removing the depletion-antibody-labeled cells from the heterogeneous immune cell population by adding affinity beads capable of specifically binding the tag to the heterogeneous immune cell population, thereby producing an enriched population of B cells;
(c) co-encapsulating the enriched population of B cells into microfluidic droplets together with:
(A):
(i) antigen-coated beads; or
(ii) antigen-expressing reporter cells; and
with at least one detection antibody capable of binding immunoglobulin secreted by the enriched population of B cells; or
(B):
(iii) a labeled antigen and immunoglobulin-capture antibody-coated beads; and
optionally, with at least one detection antibody capable of binding immunoglobulin secreted by the enriched population of B cells;
(d) incubating the droplets to allow antibody secretion;
(e) detecting droplets exhibiting a localized signal indicative of antigen-specific antibody binding;
(f) sorting positive droplets; and
(g) recovering antibody-secreting cells from the positive droplets.

2. The method of claim 1, wherein the tag is biotin, FITC, PE, APC, Cy5, an HA-tag, a FLAG-tag, a His-tag, a GST-tag, a SNAP-tag, a CLIP-tag, DIG, DNP, or a derivative thereof.

3. The method of claim 1 or 2, wherein the affinity beads comprise anti-biotin beads, anti-fluorophore beads, anti-tag beads, streptavidin beads, avidin beads, Ni-NTA beads, glutathione beads, or a combination thereof; or wherein the affinity beads are 0.1-10 µm magnetic particles coated with an anti-tag antibody, an affinity ligand, a peptide binder, a metal, a polymer, or a combination thereof.

4. The method of any one of claims 1-3, wherein the enriched population of B cells comprises an enriched population of plasma B cells; and/or wherein the species is rabbit, rat, hamster, bovine, ovine, canine, feline, camelid, llama, alpaca, a non-human primate, or a combination thereof.

5. The method of any one of claims 1-4, further comprising amplifying nucleic acids encoding immunoglobulin heavy and/or light chains, or a fragment thereof, from the antibody-secreting cells, optionally, wherein nucleic acids encoding immunoglobulin variable heavy and/or light chain sequences are amplified.

6. The method of any one of claims 1-5, wherein the at least one depletion antibody comprises an antibody recognizing one or more of: a T-cell marker, an NK-cell marker, a monocyte marker, a macrophage marker, a dendritic-cell marker, a granulocyte marker, or an IgM-positive naïve B-cell marker.

7. The method of any one of claims 1-6, wherein the at least one depletion antibody comprises a plurality of depletion antibodies, optionally, wherein the plurality of depletion antibodies comprises two, three, four, five, six, or all of a T-cell marker, an NK-cell marker, a monocyte marker, a macrophage marker, a dendritic-cell marker, a granulocyte marker, or an IgM-positive naïve B-cell marker.

8. The method of any one of claims 1-7, wherein:
(i) the method further comprises treating (a) the enriched B cells; and/or (b) the antigen-coated beads, the antigen-expressing reporter cells, or immunoglobulin-capture antibody-coated beads, with a blocking antibody or an FcR-blocking reagent to reduce non-specific interactions; and/or
(ii) the detection further comprises using a viability dye, and wherein droplets comprising dead cells are excluded.

9. The method of any one of claims 1-8, wherein the antigen-coated beads comprise polystyrene, silica, agarose, dextran, polymer, hydrogel, or magnetic beads; and/or wherein the antigen-coated beads have diameters of 20 nm to 50 µm; or wherein the antigen-expressing reporter cells express a membrane protein, a multi-pass receptor, a GPCR, an ion channel, a viral glycoprotein, or another conformational antigen.

10. The method of any one of claims 1-9, wherein:
(i) a droplet is classified as positive when:
(a) the detection antibody generates a signal that exceeds a predetermined threshold; and
(b) the signal generated by dead cell is at background level; and
(c) optionally, the signal generated by the blocking antibody is at background level; and/or
(ii) the sorting is performed by dielectrophoresis, acoustics, pneumatic valves, mechanical deflection, optical sorting, or surface-energy-based sorting.

11. The method of any one of claims 1-10, further comprising amplifying nucleic acids encoding immunoglobulin heavy and/or light chains, or a fragment thereof, from the antibody-secreting cells, and optionally further comprising sequencing the amplified nucleic acids.

12. A method for enriching a population of B cells, the method comprising:
(a) contacting a heterogeneous immune cell population with a plurality of depletion antibodies that specifically bind non-B-lineage cells of the same species, thereby producing depletion-antibody-labeled cells, wherein the plurality of depletion of antibodies are conjugated to a tag, and wherein the tag is biotin, fluorophore, a peptide tag, an affinity tag, a chemical label, or a combination thereof; and
(b) removing the depletion-antibody-labeled cells from the heterogeneous immune cell population by adding affinity beads capable of specifically binding the tag to the heterogeneous immune cell population, thereby producing an enriched population of B cells,
wherein the plurality of antibodies targeting T cells, monocytes, DCs, macrophage, IgM⁺ naïve B cells, and IgD⁺ B cells.

13. The method of claim 12, wherein:
(a) the plurality of antibodies comprises an anti-T lymphocyte antibody, an anti-CD4 antibody, an anti-CD8 antibody, an anti-CD11b antibody, an anti-IgM antibody, an anti-CD11c antibody, an anti-IgD antibody, and an anti-CD123 antibody;
(b) the tag is biotin, FITC, PE, APC, Cy5, an HA-tag, a FLAG-tag, a His-tag, a GST-tag, a SNAP-tag, a CLIP-tag, DIG, DNP, or a derivative thereof; and/or
(c) the affinity beads comprise anti-biotin beads, anti-fluorophore beads, anti-tag beads, streptavidin beads, avidin beads, Ni-NTA beads, glutathione beads, or a combination thereof.

14. A method for identifying an antigen-specific antibody-secreting cell, the method comprising:
(a) providing microfluidic droplets co-encapsulated with a population of B cells together with:
(A):
(i) antigen-coated beads; or
(ii) antigen-expressing reporter cells; and
with at least one detection antibody capable of binding immunoglobulin secreted by the population of B cells, or
(B):
(iii) an antigen with immunoglobulin-capture antibody-coated beads; and optionally, with at least one detection antibody capable of binding
immunoglobulin secreted by the population of B cells,
wherein the B cells, the antigen-coated beads, the antigen-expressing reporter cells, and/or the immunoglobulin-capture antibody-coated beads, have been treated with a blocking antibody or an FcR-blocking reagent to reduce non-specific interactions;
(b) incubating the droplets to allow antibody secretion;
(c) detecting droplets exhibiting a localized fluorescence signal indicative of antigen-specific antibody binding;
(d) sorting positive droplets; and
(e) recovering antibody-secreting cells from the positive droplets.

15. The method of claim 14, wherein:
(i) the detection further comprises using a viability dye, and wherein droplets comprising dead cells are excluded;
(ii) a droplet is classified as positive when:
(a) the detection antibody generates a signal that exceeds a predetermined threshold; and
(b) the signal generated by dead cell is at background level; and
(c) optionally, the signal generated by the blocking antibody is at background level.
(iii) the method further comprises amplifying nucleic acids encoding immunoglobulin heavy and/or light chains, or a fragment thereof, from the antibody-secreting cells; and/or
(iv) the method further comprises sequencing the amplified nucleic acid.
